# EUROPEAN PATENT APPLICATION

(11) **EP 4 813 445 A1**
(43) Date of publication of application: **30.09.2026**
(21) Application number: 26167923.7
(22) Date of filing: 26.03.2026
(51) Int. Cl.: A61N 1/372, A61N 1/375

(54) **LOADING TOOL FOR A BIOSTIMULATOR**

(30) Priority: 28.03.2025 US 202563780100 P; 25.03.2026 US 202619578732
(71) Applicant: Pacesetter, Inc., Sylmar, CA 91342 (US)
(72) Inventor: VICTORINE, Keith, Sylmar, CA 91342 (US); KWON, Daniel, Sylmar, CA 91342 (US); NYCUM, Caitlin, Sylmar, CA 91342 (US); ZHANG, Kai, Sylmar, CA 91342 (US)
(74) Representative: Barker Brettell Sweden AB

(57) **Abstract**

A loading tool (904) for loading a biostimulator (100) onto a biostimulator transport system (300) is described. The loading tool (904) includes a body portion (906) having a biostimulator volume (1016) to receive the biostimulator (100), and a receptacle portion (908) extending from the body portion (906). The receptacle portion (908) has a receptacle wall (912) extending around a receptacle volume (910) to receive the biostimulator transport system (300). The receptacle volume (910) is in fluid communication with the biostimulator volume (1016). A loading slot (914) extends laterally through the receptacle wall (912) into the receptacle volume (910). Other embodiments are also described and claimed.

## Description

### TECHNICAL FIELD

The present disclosure relates to biostimulators and related delivery systems and methods. More specifically, the present disclosure relates to devices and methods for loading a biostimulator onto a biostimulator transport system.

### BACKGROUND

Artificial pacemakers provide an electrical stimulation to the heart to perform cardiac pacing when a conduction system of the heart fails to naturally provide synchronized atrial and ventricular contractions at rates and intervals sufficient for a patient's health. Such antibradycardial pacing provides relief from symptoms and even life support for hundreds of thousands of patients.

Cardiac pacing by currently available or conventional pacemakers is usually performed by a pulse generator implanted subcutaneously or sub-muscularly in or near a patient's pectoral region. Pulse generator parameters are usually interrogated and modified by a programming device outside the body, via a loosely-coupled transformer with one inductance within the body and another outside, or via electromagnetic radiation with one antenna within the body and another outside. The pulse generator usually connects to one or more implanted leads, the distal end(s) of which contain one or more electrodes for positioning adjacent to the inside or outside wall of a cardiac chamber. The leads have an insulated electrical conductor or conductors for connecting the pulse generator to electrodes in the heart. The leads typically have lengths of 50 to 70 centimeters.

Conventional pacemakers have several drawbacks, including pulse generators that, when located subcutaneously, present a bulge in the skin that patients can find unsightly, unpleasant, or irritating. Furthermore, conventional pacemakers have complex connections to the leads that can malfunction. Other problematic aspects of conventional pacemakers relate to the separately implanted pulse generator and the pacing leads. By way of example, the pacing leads can become a site of infection and morbidity.

Many of the issues associated with conventional pacemakers are resolved by a self-contained and self-sustainable pacemaker, or so-called leadless pacemaker. Similar to active fixation implantable leads used with conventional pulse generators, leadless pacemakers are typically fixed to an intracardial implant site by an actively engaging mechanism such as a helical member that screws into the myocardium. Leadless pacemakers are typically delivered to an intracardial implant site via a delivery system including catheters, sheaths, and/or introducers. Such leadless pacemakers are typically preloaded onto the delivery system during manufacturing and then packaged and sterilized in that preloaded configuration.

### SUMMARY

Biostimulators, e.g., leadless cardiac pacemakers, that are packaged and preloaded on a delivery system can present several issues. For example, if the physician needs to implant multiple biostimulators into the patient, as may be the case in a dual chamber cardiac pacing arrangement, the physician would require two separate preloaded delivery systems to deliver both biostimulators. Also, if the biostimulator is damaged during the course of the procedure, but the delivery system is still functional, the physician would need to open a new preloaded delivery system in order to complete the procedure. Finally, sterilization cycles, shelf life, distribution, and inventory management are complicated by having the biostimulator joined to the delivery system in manufacturing. All of the forgoing result in significant waste and additional cost associated with each implantation procedure.

A loading tool for loading a biostimulator onto a biostimulator delivery system is provided. The loading tool includes a body portion having a biostimulator volume configured to receive the biostimulator. The loading tool includes a receptacle portion extending from the body portion. The receptacle portion has a receptacle wall extending around a receptacle volume configured to receive the biostimulator transport system. The receptacle volume is in fluid communication with the biostimulator volume. A loading slot extends laterally through the receptacle wall into the receptacle volume. Accordingly, the loading tool can provide an easy-to-use solution for loading a biostimulator onto a biostimulator delivery system in the clinical setting, thereby eliminating the need to preload.

A biostimulator system is provided. The biostimulator system comprises a biostimulator including a housing containing circuitry, a fixation element coupled to a distal end of the housing, and an attachment feature coupled to a proximal end of the housing. The biostimulator system also comprises a loading tool according to the embodiments. The biostimulator volume contains the biostimulator.

A method of loading a biostimulator onto a biostimulator delivery system is provided. The method includes inserting a tether of the biostimulator transport system into an attachment feature of the biostimulator. The biostimulator is disposed in a receptacle volume of a loading tool. The tether passes laterally through a loading slot into the receptacle volume. The method includes unlocking a latch of the loading tool. The latch is mounted on a first body portion of the loading tool. The latch fastens the first body portion to a second body portion. The method includes swinging the first body portion about a hinge that holds the first body portion to the second body portion to open the biostimulator volume of the loading tool. The method includes removing the loading tool from the biostimulator and the biostimulator transport system.

The above summary does not include an exhaustive list of all aspects of the present invention. It is contemplated that the invention includes all systems, devices, and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

FIG. 1 is a pictorial view of biostimulators implanted in a patient heart, in accordance with an embodiment.
FIG. 2 is a side view of a biostimulator, in accordance with an embodiment.
FIG. 3 is a perspective view of a biostimulator transport system, in accordance with an embodiment.
FIG. 4 is a perspective view of an attachment feature of a biostimulator, in accordance with an embodiment.
FIG. 5 is a side view of a tether of a biostimulator transport system being inserted into an attachment feature of a biostimulator, in accordance with an embodiment.
FIG. 6 is a perspective view of a tether of a biostimulator transport system being inserted into an attachment feature of a biostimulator, in accordance with an embodiment.
FIG. 7 is a side view of a tether of a biostimulator transport system being locked into an attachment feature of a biostimulator, in accordance with an embodiment.
FIG. 8 is a perspective view of a tether of a biostimulator transport system locked into an attachment feature of a biostimulator, in accordance with an embodiment.
FIG. 9 is a top view of a loading tool in a closed configuration, in accordance with an embodiment.
FIG. 10 is a perspective view of a loading tool in an open configuration, in accordance with an embodiment.
FIG. 11 is a perspective view of a docking cap of a biostimulator transport system inserted into a loading tool, in accordance with an embodiment.
FIG. 12 is a detail view of a friction feature of a loading tool, in accordance with an embodiment.
FIG. 13 is a flowchart of a method of loading a biostimulator onto a biostimulator transport system, in accordance with an embodiment.
FIG. 14 is a perspective view of a loading tool in a closed configuration, in accordance with an embodiment.
FIG. 15 is a perspective view of a receptacle portion of a loading tool, in accordance with an embodiment.
FIG. 16 is a top view of a receptacle portion of a loading tool, in accordance with an embodiment.
FIG. 17 is a perspective sectional view of a receptacle portion of a loading tool, in accordance with an embodiment.
FIG. 18 is a perspective sectional view of a receptacle portion of a loading tool, in accordance with an embodiment.
FIG. 19 is a perspective view of a loading tool in a closed configuration, in accordance with an embodiment.
FIG. 20 is a perspective view of a receptacle portion of a loading tool, in accordance with an embodiment.
FIG. 21 is a top view of a receptacle portion of a loading tool, in accordance with an embodiment.
FIG. 22 is a perspective sectional view of a receptacle portion of a loading tool, in accordance with an embodiment.
FIG. 23 is a perspective sectional view of a receptacle portion of a loading tool, in accordance with an embodiment.
FIG. 24 is a perspective view of an attachment feature of a biostimulator, in accordance with an embodiment.
FIGS. 25-28 are perspective sectional views of an attachment feature of a biostimulator, in accordance with an embodiment.

### DETAILED DESCRIPTION

Embodiments describe a loading tool and method for loading a biostimulator, such as a leadless cardiac pacemaker, onto a biostimulator transport system, such as a delivery system. The loading tool can be used in a catheterization laboratory to load the biostimulator onto the biostimulator transport system prior to implantation into a patient. The loading tool, however, may be used in other applications, such as in a manufacturing setting to load the biostimulator onto the biostimulator transport system prior to shipment to the customer.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "proximal" may indicate a first direction along a longitudinal axis of a loading tool. Similarly, "distal" may indicate a second direction opposite to the first direction. Such terms are provided to establish relative frames of reference, however, and are not intended to limit the use or orientation of a loading tool to a specific configuration described in the various embodiments below.

In an aspect, a loading tool can include a loading slot extending laterally through a receptacle wall into a receptacle volume in which a biostimulator may be located. The loading slot can accommodate passage of a portion of a biostimulator transport system that must engage the biostimulator. For example, a ball and/or a tether of a coupling mechanism of the biostimulator transport system can pass through the loading slot to engage an attachment feature of the biostimulator within the biostimulator volume. The loading can occur while the biostimulator remains protected by a receptacle wall surrounding the biostimulator volume. Furthermore, the loading slot can guide insertion of the biostimulator transport system to execute the loading process using a ball-cone catheter configuration. Accordingly, the loading tool can protect the biostimulator during the unpackaging process, e.g., from scratches or impacts, and can assist a user in loading the biostimulator onto the biostimulator transport system prior to delivering the biostimulator to a target anatomy.

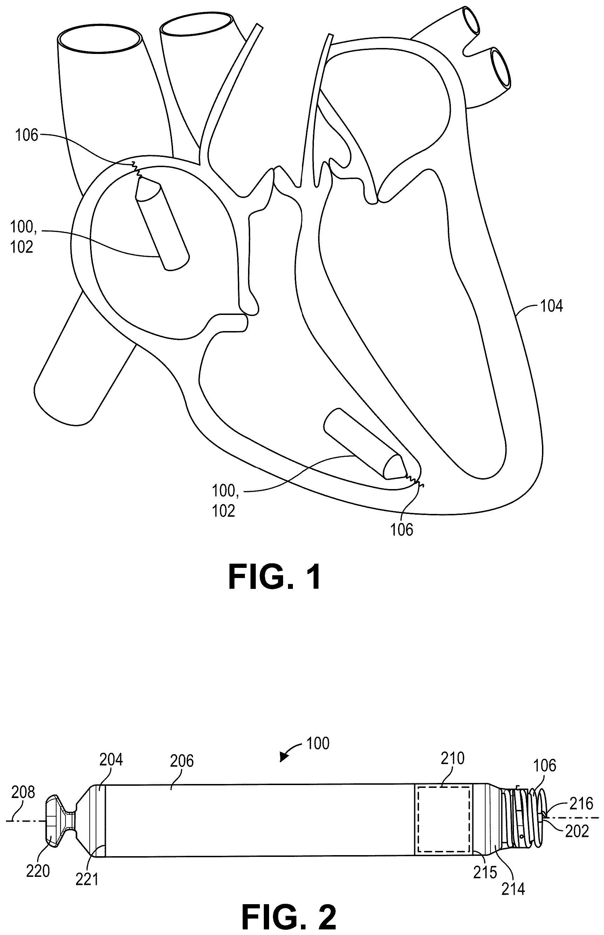

Referring to FIG. 1, a pictorial view of biostimulators 100 implanted in a patient heart is shown in accordance with an embodiment. A biostimulator system, e.g., useful for cardiac pacing, includes one or more biostimulators 100. The biostimulators 100 can be implanted in the patient heart 104, and can be leadless, and thus may be leadless cardiac pacemakers 102.

Each biostimulator 100 can be placed in a cardiac chamber, such as a right atrium and/or a right ventricle of the patient heart 104, or attached to an inside or outside of the cardiac chamber. The biostimulator 100 can be implanted inside the heart 104 using a catheter (such as the biostimulator transport system described below), which can enter the heart through a superior or inferior vena cava. The catheter can guide the biostimulator 100 into the right atrium and/or ventricle to a target location of the target anatomy. For example, the biostimulator can be delivered to an implantation site having cardiac tissue.

Attachment of the biostimulator 100 to the cardiac tissue can be accomplished via one or more fixation elements 106, such as helical anchors or curved splines or tines, that engage and anchor the biostimulator 100 in a target anatomy. In a particular embodiment, the biostimulator 100, such as in the form of a leadless cardiac pacemaker 102, can use two or more electrodes located on or within a housing of the leadless cardiac pacemaker 102 for pacing the cardiac chamber upon receiving a triggering signal from internal circuitry and/or from at least one other device within the body.

Referring to FIG. 2, a side view of a biostimulator 100 is shown in accordance with an embodiment. The biostimulator 100 can be a leadless cardiac pacemaker 102 that can perform cardiac pacing and that has many of the advantages of conventional cardiac pacemakers while extending performance, functionality, and operating characteristics. The biostimulator 100 can have two or more electrodes, e.g., a distal electrode 202 and a proximal electrode 204, located within, on, or near a biostimulator housing 206 of the biostimulator 100. In an embodiment, one or more of the fixation elements 106 form a portion of the distal electrode 202. The electrodes 202, 204 can deliver pacing pulses to muscle of the cardiac chamber, and optionally, can sense electrical activity from the muscle. The electrodes 202, 204 may also communicate bidirectionally with at least one other device within or outside the body.

In an embodiment, the biostimulator housing 206 has a longitudinal axis 208, and the distal electrode 202 can be a distal pacing electrode mounted on the biostimulator housing 206 along the longitudinal axis 208. The biostimulator housing 206 can contain a primary battery to provide power for pacing, sensing, and communication, which may include, for example, bidirectional communication. The biostimulator housing 206 can optionally contain an electronics compartment 210 to hold circuitry adapted for different functionality. For example, the electronics compartment 210 can be located on a longitudinal axis 208 of the biostimulator 100 and can contain circuits for sensing cardiac activity from the electrodes 202, 204, circuits for receiving information from at least one other device via the electrodes 202, 204, circuits for generating pacing pulses for delivery via the electrodes 202, 204, or other circuitry. The electronics compartment 210 may contain circuits for transmitting information to at least one other device via the electrodes 202, 204 and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers 102 or other leadless biostimulators 100 can be fixed to an intracardial implant site by one or more actively engaging mechanism or element or fixation mechanism or element 106. The fixation mechanism or element 106 can include a screw or helical member that screws into the myocardium. Alternatively, the fixation mechanism or element 106 can include tines that pierce and curve outward to grip the myocardium.

In an embodiment, the biostimulator 100 includes the fixation element 106 coupled to the biostimulator housing 206. The fixation element 106 can be a helical element to screw into target tissue. More particularly, the fixation element 106 can extend helically from a flange 214 of the biostimulator 100, which is mounted on the biostimulator housing 206, to a distal tip at a helix distal end 216. Accordingly, the fixation element 106 can be coupled to a distal end 215 of the housing 206.

The helix distal end 216 can be located distal to the distal electrode 202 (a centrally located electrode). Accordingly, when the biostimulator 100 contacts the target tissue, the distal tip can pierce the tissue and the housing 206 can be rotated to screw the outer fixation element 106 into the target tissue to pull the distal electrode 202 into contact with the tissue.

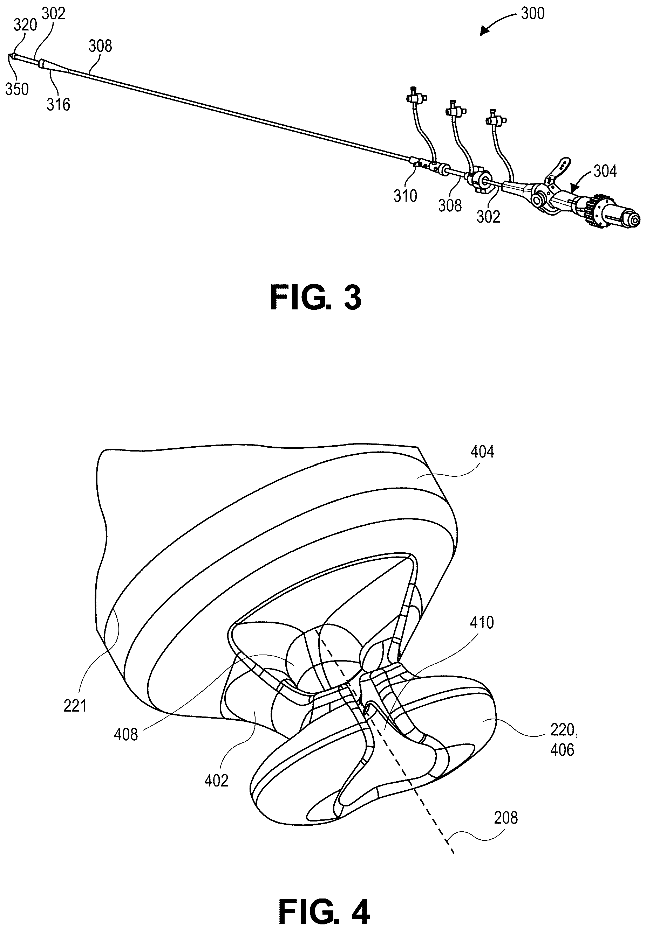

The biostimulator 100 includes an attachment feature 220. The attachment feature 220 generally facilitates coupling of the biostimulator 100 to a biostimulator transport system (FIG. 3). More particularly, the attachment feature 220 can include features that can be engaged and retained by a release mechanism 350, also referred to as coupling mechanism herein, of the biostimulator transport system 300, as described below. In an embodiment, the attachment feature 220 is coupled to a proximal end 221 of the housing 206. For example, the attachment feature 220 can be welded to the proximal end 221 to connect the attachment feature 220 to the housing 206 physically and/or electrically.

Referring to FIG. 3, a perspective view of a biostimulator transport system 300 is shown in accordance with an embodiment. Leadless pacemakers 102 or other biostimulators 100 can be delivered to and retrieved from a patient anatomy using a biostimulator transport system 300. In some implementations, the biostimulator transport system 300 is a delivery system for delivering the biostimulator 100 to the target tissue. In some implementations, the biostimulator transport system 300 is a retrieval system for retrieving the biostimulator 100 from the target tissue. As described below, the biostimulator transport system 300 can include a release or coupling mechanism 350 to retain the biostimulator 100 in an unreleased state and to transition into a released state to release the biostimulator 100 into the target anatomy.

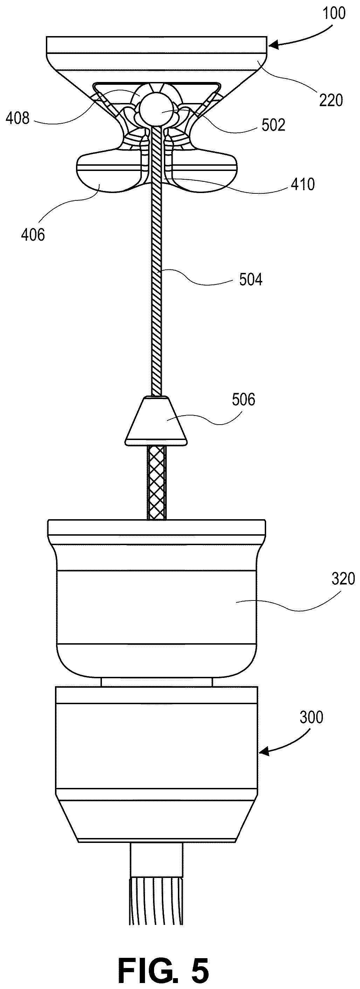

The biostimulator transport system 300 can include an elongated catheter 302 extending distally from a handle 304 to a distal catheter end. The elongated catheter 302 can be a deflectable catheter, and an operator can use the handle 304 to steer the distal catheter end in the patient. In an embodiment, the biostimulator transport system 300 includes a guide catheter 308 mounted on the elongated catheter 302. The guide catheter 308 can be slidably disposed on the elongated catheter 302 such that a distal portion of the guide catheter 308 can slide distally over the distal catheter end of the elongated catheter 302 and/or the biostimulator 100, which may be mounted on the distal catheter end (e.g., FIG. 5). Similarly, the biostimulator transport system 300 can include an introducer hub assembly 310 mounted on the guide catheter 308. The introducer hub assembly 310 can be slidably disposed on the guide catheter 308 such that a distal portion of the introducer hub assembly 310 can slide distally over the distal catheter end of the elongated catheter 302 and/or the distal portion of the guide catheter 308. More particularly, the introducer hub assembly 310 can be inserted into an access sheath to gain access to the patient vasculature, and after access is established, the distal portion of the guide catheter 308 and/or the distal catheter end of the elongated catheter 302 can be advanced through the access sheath into the patient.

The distal catheter end of the elongated catheter 302 may be selectively connectable to the biostimulator 100. More particularly, the biostimulator 100 can be mounted on the distal catheter end of the elongated catheter 302. The biostimulator 100 can be protected by a protective sheath 316 of the distal portion of the guide catheter 308 during delivery and/or retrieval of the biostimulator 100 from the patient. Accordingly, the biostimulator 100 can be advanced into the patient along with the distal catheter end.

The biostimulator transport system 300 can be used to implant one or more biostimulators 100 within an atrium and/or a ventricle of a heart 104 of the patient. Implantation of each biostimulator 100 may be achieved, in part, by endocardial insertion of the biostimulators 100. For example, the elongated catheter 302 of the biostimulator transport system 300 can include a torque shaft, within an outer member of the elongated catheter 302, coupled to a docking cap 320. The docking cap 320 can have a docking cavity to receive the attachment feature 220 of the biostimulator 100. The torque shaft can be torqueable, and rotation of the torque shaft can rotate the docking cap 320, which can impart rotation to the attachment feature 220. Accordingly, torque can be transmitted through the torque shaft to rotate the biostimulator 100 in a first direction, e.g., clockwise. Rotating the biostimulator 100 when the fixation element 106 is in contact with the heart tissue can cause the fixation element 106 to screw into the heart tissue and affix the biostimulator 100 to the heart tissue. Similarly, removal and retrieval of the biostimulator 100 may be accomplished endocardially. For example, the torque shaft of the elongated catheter 302 can be rotated in a second direction, e.g., counterclockwise, to transmit torque through the docking cap 320 to the attachment feature 220 to disengage the biostimulator 100 from the heart tissue.

The biostimulator transport system 300 can include a release mechanism 350. The release mechanism 350 may be located at the distal catheter end. The release mechanism 350 can act as a coupling mechanism (and be so-called) to directly connect the biostimulator 100 to the biostimulator transport system 300. For example, the release mechanism 350 can include a locking ball 502 and/or tether 504, as described below, which can extend distal to the docking cap 320 to engage the biostimulator 100. The release mechanism 350 can engage the biostimulator 100 in the unreleased state and disengage from the biostimulator 100 in the released state. Accordingly, delivery and retrieval systems having a structure similar to that shown in FIG. 3 may be used to deliver and/or retrieve the biostimulator 100 from a target anatomy.

Referring to FIG. 4, a perspective view of an attachment feature 220 of a biostimulator 100 is shown in accordance with an embodiment. The attachment feature 220 can be shaped to receive the release mechanism 350, e.g., the locking ball and/or tether, to facilitate engagement between the biostimulator 100 and the biostimulator transport system 300. In an embodiment, the attachment feature 220 is mounted on the proximal end 221 of the housing 206 and extends proximally along the longitudinal axis 208. During delivery or retrieval, the longitudinal axis 208 can align with the central axis of biostimulator transport system 300. For example, the central axis can extend through a docking cavity of the docking cap 320, which can receive the attachment feature 220. The attachment feature 220 can also include features to allow components of the biostimulator transport system 300, such as the locking ball and/or tether, to engage and connect to the biostimulator 100.

The attachment feature 220 can include a neck 402 that extends proximally from the housing 206. More particularly, the neck 402 can be a portion of the attachment feature 220 that extends from a base 404 at the proximal end 221 to a head 406 of the attachment feature 220 at a most proximal location. The base 404, neck 402, and head 406 can combine to form an overall volume of the attachment feature 220. Within such volume there may be one or more channels to receive components of the biostimulator transport system 300, as described below. The channel(s) can include a locking channel 408 within which the locking ball and/or tether can fit within.

In an embodiment, the attachment feature 220 includes the locking channel 408 extending longitudinally through the neck 402. A cross-sectional area of the locking channel 408 can vary in the longitudinal direction. For example, the locking channel 408 may be wider at a distal location that receives the ball of the biostimulator transport system 300. Here, "wider" may be relative to a portion of the central channel that is proximal to the distal recess. The distal recess opens radially outward to receive the ball. For example, the proximal portion of the central channel may be a portion within the head 406 of the attachment feature 220, which receives the tether of the biostimulator transport system 300. The portions of the locking channel 408 may therefore have respective cross-sectional areas corresponding to the coupling components of the biostimulator transport system 300 that the portions receive.

The attachment feature 220 can include a lateral slot 410 extending laterally, e.g., transverse to the longitudinal direction, to the locking channel 408. The lateral slot 410 can, for example, extend laterally through the neck 402. The slot 410 may also extend laterally through the head 406 and/or the base 404. Accordingly, the lateral slot 410 can form a window into the locking channel 408 that extends along the longitudinal axis 208. More particularly, the lateral slot 410 can provide a lateral opening through which the locking ball and/or tether can pass when the biostimulator transport system 300 is being loaded into the biostimulator 100.

Referring to FIG. 5, a side view of a tether 504 of a biostimulator transport system 300 being inserted into an attachment feature 220 of a biostimulator 100 is shown in accordance with an embodiment. The loading of the biostimulator transport system 300 into the biostimulator 100 is evident from a ball 502 of the biostimulator transport system 300 being inserted into the locking channel 408 to engage the attachment feature 220. Similarly, a tether 504 of the biostimulator transport system 300 can be loaded into the locking channel 408 by passing through the lateral slot 410. The ball 502 can include a metallic sphere. The ball 502 can be mounted on or at a distal end of the tether 504. For example, the ball 502 can be welded onto the end of the tether 504. The tether 504 may include a braided wire, e.g., a cable. Accordingly, the ball 502 and tether 504 may be secured to each other to form a structure that can engage and tether the attachment feature 220 to the biostimulator transport system 300. When the ball 502 and tether 504 are allowed to pass laterally through the lateral slot 410 into the locking channel 408, the biostimulator 100 can be loaded onto the biostimulator transport system 300.

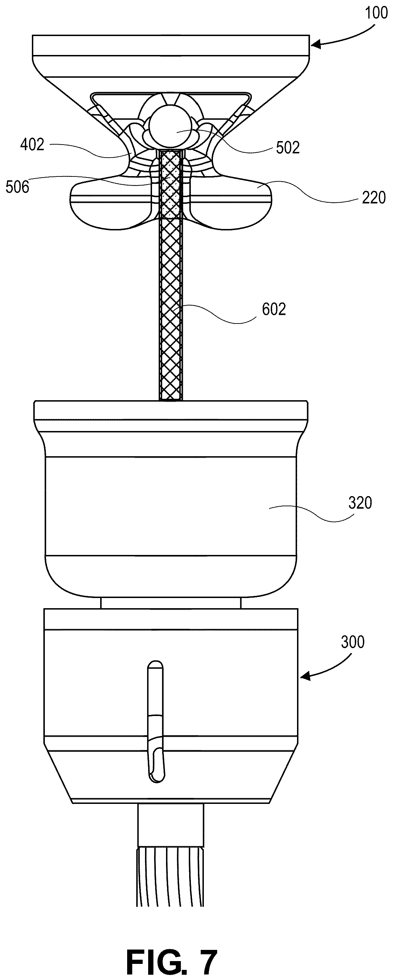

The biostimulator transport system 300 can include additional coupling mechanism 350 (e.g., release mechanism) components that engage portions of the attachment feature 220 and locking channel 408. For example, the biostimulator transport system 300 can include a conical insert 506 that slides over the tether 504 to slide into the locking channel 408 at a proximal end of the head 406 of the attachment feature 220. The conical insert 506 can include a rubber cone stopper. In an embodiment, the conical insert 506 can be formed from stainless steel or another hard material to prevent the conical insert 506 from behaving like a stopper that lodges in the attachment feature 220. The locking channel 408 at the proximal end can flare outward and, thus, can receive a distal tapering surface of the conical insert 506 when it slides distally over the tether 504, as described below with respect to FIGS. 7-8.

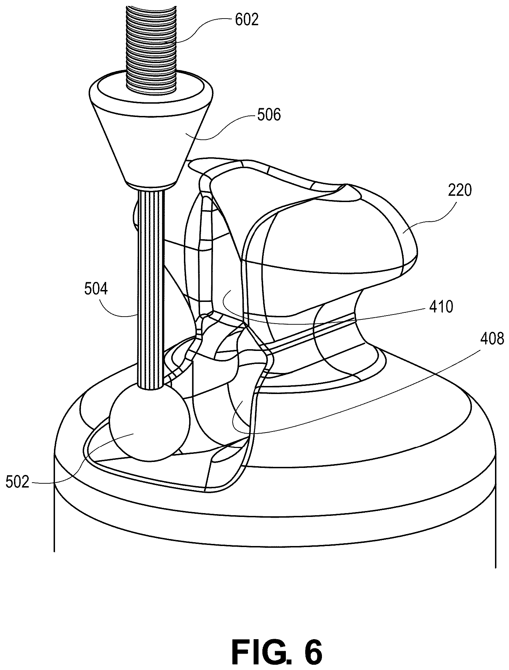

Referring to FIG. 6, a perspective view of a tether 504 of a biostimulator transport system 300 being inserted into an attachment feature 220 of a biostimulator 100 is shown in accordance with an embodiment. A locking tube 602 can support the conical insert 506, which provides a locking end of a tether assembly. The locking tube 602 can include a braided wire tube. The locking tube 602 can be retracted relative to the locking end to create a space between the conical insert 506 and the ball 502. The space is traversed by the tether 504. More particularly, the tether 504 can extend distally through the locking tube 602 to the ball 502, and a length of the tether 504 can be exposed distally from the conical insert 506. The length of the exposed tether 504 can be greater than a distance between the distal portion of the locking channel 408, which receives the ball 502, and a proximal end of the head 406. For example, the distance in the default position may be approximately 8 mm. Accordingly, the ball 502 can be laterally loaded into the locking channel 408 and the tether 504 can be laterally loaded through the lateral slot 410 to position the tether assembly within the attachment feature 220 with the conical insert 506 located proximal from the attachment feature 220 along the longitudinal axis 208.

Referring to FIG. 7, a side view of a tether of a biostimulator transport system 300 being locked into an attachment feature 220 of a biostimulator 100 is shown in accordance with an embodiment. Portions of the head 406 or the neck 402 of the attachment feature 220 can be wedged between the ball 502 and the conical insert 506 to affix the attachment feature 220 to the biostimulator transport system 300. When the ball 502 and the tether 504 are located within the locking channel 408, the locking tube 602 can be advanced distally over the tether 504 to move the conical insert 506 toward the ball 502, toward a final position. The conical insert 506 can slide into the outward flaring portion of the locking channel 408 and can squeeze the neck 402 against the ball 502 in the final position. Accordingly, the attachment feature 220 can become locked to the biostimulator transport system 300.

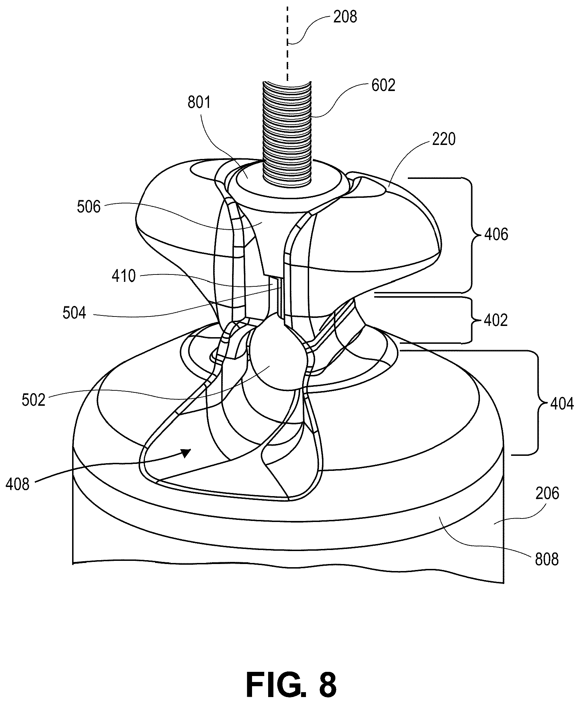

Referring to FIG. 8, a perspective view of a tether 504 of a biostimulator transport system 300 locked into an attachment feature 220 of a biostimulator 100 is shown in accordance with an embodiment. The conical insert 506 is shown inserted into the locking channel 408. In an embodiment, the conical insert 506 has a conical profile. More particularly, a proximal end 801 of the conical insert 506 can be wider than a distal end of conical insert 506. The conical profile can therefore taper from the proximal end 801 to the distal end. Similarly, the locking channel 408 may have a tapered section to receive the conical insert 506. For example, the conical feature can be tapered to engage the tapered section such that an outward facing conical surface of the conical insert 506 opposes an inward facing surface surrounding the locking channel 408. Accordingly, the conical profile can support and stabilize the locking tube 602 relative to the attachment feature 220 when the conical insert 506 is inserted into the locking channel 408.

In alternative embodiments, the conical insert 506 can have different geometries. For example, the conical insert 506 can include hemispherical, oval, or pyramidal shapes. A pyramidal conical insert can have three or four sides. These alternative geometries can provide different mechanical properties for engaging the locking channel 408 and securing the attachment feature 220 to the biostimulator transport system 300.

The attachment feature 220 may be segmented into portions. The portions can be arranged along the longitudinal axis 208 of the attachment feature 220. In an embodiment, the portions of the attachment feature 220, which are also referred to above, include a base 404, a neck 402, and a head 406. Such segments may, however, be segmented for purposes of description. For example, the head 406 may be a proximal most portion of the attachment feature 220, the base 404 may be a distal most portion of the attachment feature 220, and the neck 402 may be a portion of the attachment feature 220 intermediate between the head 406 and the base 404, and having a cross-sectional dimension that is smaller than both the head 406 and the base 404.

The base 404 may mount on a proximal end 221 of the housing 206. Accordingly, the base 404 may have an outer dimension, e.g., at a proximal lip 808, that is equal to an outer dimension of the housing 206. By contrast, the neck 402 may be narrower than the base 404. For example, the base 404 can taper inward from a distal most edge of the lip 808 at the housing 206 to a proximal most edge at the neck 402. The head 406 may extend proximally from the neck 402 and taper outward to a larger dimension. For example, the head 406 may include an anvil shape that widens to allow a retrieval snare to grasp the attachment feature 220 around the neck 402 distal to the head 406.

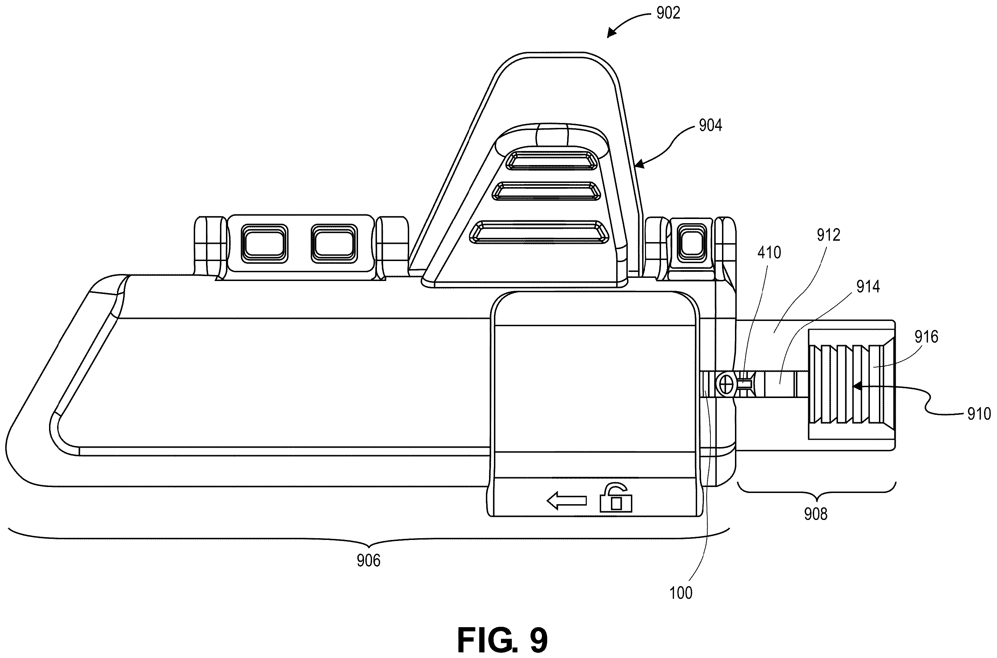

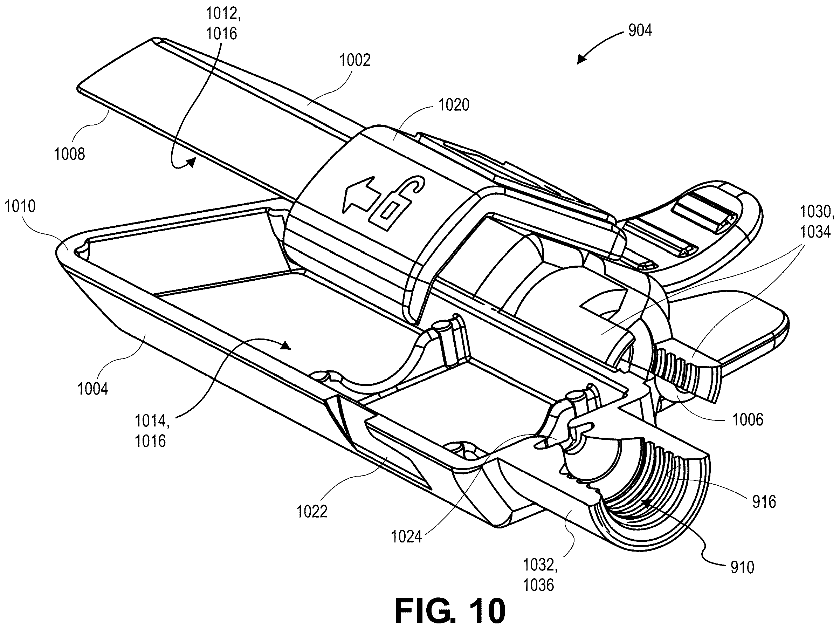

Referring to FIG. 9, a top view of a loading tool 904 in a closed configuration is shown in accordance with an embodiment. A biostimulator system can include the biostimulator 100, as described above, loaded into or stored within a loading tool 904. More particularly, the loading tool 904 can include a body portion 906 having a biostimulator volume (FIG. 10) containing the biostimulator 100. The loading tool 904 may be used to load the biostimulator 100 onto the biostimulator transport system 300. More particularly, the loading tool 904 can function as a case or a container to protect the biostimulator 100 and help guide the coupling mechanism 350 into the biostimulator 100 when the physician loads the biostimulator 100 onto the biostimulator transport system 300 using the ball 502 and tether mechanism. Accordingly, the loading tool 904 can include a portion, e.g., a receptacle portion 908, to receive the coupling mechanism 350 of the biostimulator transport system 300.

The receptacle portion 908 can extend from the body portion 906. Whereas the body portion 906 can include the biostimulator volume to receive the biostimulator 100, the receptacle portion 908 can include a receptacle volume 910 to receive the biostimulator transport system 300. More particularly, the receptacle volume 910 can receive a distal portion of the biostimulator transport system 300, such as the release mechanism 350 and/or the docking cap 320.

The portions of the loading tool 904 can include surfaces that define the tool structure. For example, walls of the loading tool 904 can extend around cavities, recesses, or channels of the loading tool 904 to define the areas within which the biostimulator 100 or the biostimulator transport system 300 may be inserted. In an embodiment, the receptacle portion 908 has a receptacle wall 912 extending around the receptacle volume 910. The receptacle wall 912 can have a thickness that provides some rigidity and strength to the receptacle portion 908. More particularly, the receptacle wall 912 can form a proximally-extending protrusion from the body portion 906, which may receive and support the release mechanism 350 of the biostimulator transport system 300.

In an embodiment, the receptacle portion 908 includes one or more slots to allow the coupling mechanism 350 of the biostimulator transport system 300 to pass through the receptacle wall 912 into the receptacle volume 910. For example, the loading tool 904 can include a loading slot 914 extending laterally through the receptacle wall 912 into the receptacle volume 910. As described below, the loading slot 914 can allow the biostimulator transport system 300, e.g., the ball 502 or tether 504, to pass laterally inward through the loading slot 914 into the volume within the receptacle portion 908. For example, the loading slot 914 can be aligned with the lateral slot 410 of the attachment feature 220. Accordingly, the ball 502 or tether 504 can pass into the attachment feature 220 within a distal portion of the loading slot 914. More particularly, the loading slot 914 can allow passage of the tether 504 of the biostimulator transport system 300 into the lateral slot 410 of the attachment feature 220. Similarly, the docking cap 320 can pass into a proximal portion of the loading slot 914 to rest against an interior surface 916 of the receptacle wall 912 proximal to the attachment feature 220. The interior surface 916 of the receptacle wall 912 can surround the receptacle volume 910. As described below, when the ball 502 fits into the attachment feature 220 and the conical insert 506 moves distally to squeeze the head 406, a robust lock between the biostimulator 100 and the biostimulator transport system 300 can be formed.

Referring to FIG. 10, a perspective view of a loading tool 904 in an open configuration is shown in accordance with an embodiment. As described above, one function of the loading tool 904 is to provide a package for the biostimulator 100. That is, the loading tool 904 can serve as a case or container to house the biostimulator 100 prior to clinical use.

A biostimulator system 902 includes a loading tool 904 for loading the biostimulator 100 onto a biostimulator delivery system 300. The biostimulator 100 can be mounted on and/or in the loading tool 904. For example, the loading tool 904 can include a first body portion 1002 movably connected to a second body portion 1004, and the biostimulator 100 can be mounted on one of the body portions 1002, 1004 in an open configuration or mounted in a volume defined by a combination of both body portions 1002, 1004 in a closed configuration. Accordingly, the biostimulator 100 can be packaged or stored in the loading tool 904 (FIG. 9).

The loading tool 904 can include walls that protect the biostimulator 100 and prevent damage to the biostimulator 100 from being inflicted by impacts during delivery. For example, in the event that a user drops the biostimulator 100 after removing the loading tool 904 from packaging, the biostimulator 100 can remain safe due to the protection provided by the loading tool barrier. In an embodiment, the loading tool 904 includes the body portion to surround the biostimulator 100.

The loading tool 904 may have a clamshell design to allow the biostimulator 100 to be loaded into the body portion 906. More particularly, the body portion 906 can include a first body portion 1002 hinged to a second body portion 1004, and the body portions can move relative to each other about the hinge 1006 between an open configuration (FIG. 10) and a closed configuration (FIG. 9). The biostimulator 100 can be loaded into one of the body portions 1002, 1004 when the loading tool 904 is in the open configuration, and then contained by the body portions 1002, 1004 by closing the body portions 1002, 1004 against each other.

In an embodiment, the second body portion 1004 is hinged to the first body portion 1002. For example, the first body portion 1002 may be pivotally connected to the second body portion 1004 by a hinge 1006. The hinge 1006 can include a pinned connection between hinge elements, or the hinge 1006 may be a living hinge, such as a thin strap of material having ends connected to each of the first body portion 1002 and the second body portion 1004. Accordingly, the hinge 1006 may be integrally formed with the body portions, and the loading tool 904 may have a monolithic construction.

Each of the body portions 1002, 1004 may be further segmented, either physically or conceptually, into several subportions. For example, each body portion 1002, 1004 may have a distal subportion and a proximal subportion proximal to the distal subportion. The body portions 1002, 1004, and the respective subportions, can include surfaces that mate in the closed configuration. For example, the first body portion 1002 can include a first face 1008, and the second body portion 1004 can include a second face 1010. When the first body portion 1002 is pivoted about the hinge 1006 from the open configuration to the closed configuration, the first face 1008 can appose the second face 1010.

One or more of the body portions 1002, 1004 can be formed from a rigid polymer, such as Copolyester Eastman Tritan MX731, which may have sufficient transparency to view a cavity housed within the portions. For example, each body portion 1002, 1004 can include one or more recesses in the subportions, and the recesses can combine to form the cavity that holds the biostimulator 100, which is visible through the body portion walls. A first recess can be formed in the first body portion 1002. The first recess can project into the first face 1008 and extend from a distal end of the body portion 1002 to a proximal end of the body portion 1002. Thus, the first recess can include recess segments in the distal subportion and the proximal subportion of the first body portion 1002. Similarly, a second recess can be formed in the second body portion 1004. The second recess can project into the second face 1010 and extend from a distal end of the body portion 1004 to a proximal end of the body portion 1004. Thus, the second recess can include recess segments in the distal subportion and the proximal subportion of the second body portion 1004.

When the first face 1008 of the first body portion 1002 is apposed to the second face 1010 of the second body portion 1004, e.g., in the closed configuration of the loading tool 904, the first recess in the first body portion 1002 and the second recess in the second body portion 1004 can combine to form a biostimulator volume 1016. In an embodiment, the biostimulator volume 1016 can be further segmented into subvolumes having respective functions. For example, the distal subportion can be configured to receive the biostimulator 100, and the proximal subportion can include the receptacle volume 910 configured to receive a portion of the biostimulator transport system 300, such as the docking cap 320.

The biostimulator volume 1016 and respective subvolumes defined by the internal surfaces of the loading tool body portions 1002, 1004 may be sized and configured to contain and hold the biostimulator 100. For example, the inner surfaces of the distal subportions of first body portion 1002 and second body portion 1004, which define the recesses, may be configured to conform to an outer surface of the housing 206 and the attachment feature 220 of the biostimulator 100. The inner surfaces defining the recesses can be configured to receive the biostimulator 100 and to facilitate joining the biostimulator to the biostimulator transport system 300.

A latch 1020 may be slidably mounted on the first body portion 1002. The latch 1020 may, for example, be formed from a rigid polymer such as Acetal Americhem Delrin, having sufficient strength to lock the two clamshell portions, e.g., the first body portion 1002 and the second body portion 1004, together. By way of example, the latch 1020 can have a latch tab (not shown) that can insert into a latch slot 1022 of the second body portion 1004. The latch prong can be retained in the latch slot 1022 to hold the first body portion 1002 against the second body portion 1004 in the closed configuration (FIG. 9).

In an embodiment, the biostimulator volume 1016 is in fluid communication with the receptacle volume 910. For example, the proximal subportion of the biostimulator volume 1016 can include a head volume 1024, which is sized and configured to receive the head 406 of the attachment feature 220. The head volume 1024 can open longitudinally, directly to the receptacle volume 910 through a central aperture that permits passage of the tether 504 along the longitudinal axis 208 between the receptacle volume 910 and the head volume 1024. Accordingly, the biostimulator volume 1016, and subportions thereof, can be connected to the receptacle volume 910.

The receptacle portion 908, which can include the tool subportion proximal to the body portion 906, may split into two segments along a transverse split plane. For example, the receptacle portion 908 can include a top wall segment 1030, above the split plane, and a bottom wall segment 1032, below the split plane. Here, "above" and "below" refers to opposite sides of the split plane.

The top wall segment 1030 and the bottom wall segment 1032 can combine to form the receptacle volume 910 within which the coupling mechanism 350 inserts into during the biostimulator loading process. In an embodiment, one of the segments includes a lateral channel through which the coupling mechanism 350 can pass, and the other segment is solid, without a lateral channel, to securely hold the coupling mechanism 350. For example, the top wall segment 1030 can have several cantilever arms 1034, separated across a lateral space such as the loading slot 914. More particularly, the cantilever arms 1034 can extend proximally at radially separated locations from each other to creates two prongs between which the docking cap 320 may pass into the receptacle volume 910. By contrast, only the top wall segment 1030 may have the keyhole slot cut out for the ball-cone catheter, and the bottom wall segment 1032 may not. The bottom wall segment 1032 can have a semi-cylindrical wall 1036. The semi-cylindrical wall 1036 can include a solid wall segment, which extends fully around an angular section of the docking cap 320 when the docking cap 320 is placed in the receptacle volume 910. The semi-cylindrical wall 1036 can therefore support and appose the docking cap 320 when the biostimulator 100 is loaded onto the coupling mechanism 350.

The receptacle portion 908 includes the several cantilever arms 1034 extending proximally on laterally opposite sides of the loading slot 914. When the loading tool 904 is in the open configuration, the cantilever arms 1034 can be free suspended as cantilevers from a more distal subportion of the receptacle portion 908. The semi-cylindrical wall 1036 can therefore have a semi-cylindrical volume exposed upward. In the closed configuration, however, the cantilever arms 1034 can move toward and appose the semi-cylindrical wall 1036. Accordingly, a combined receptacle portion 908 can be formed including a structure that is cylindrical or annular in shape, however, includes the loading slot 914 passing radially inward toward the receptacle volume 910.

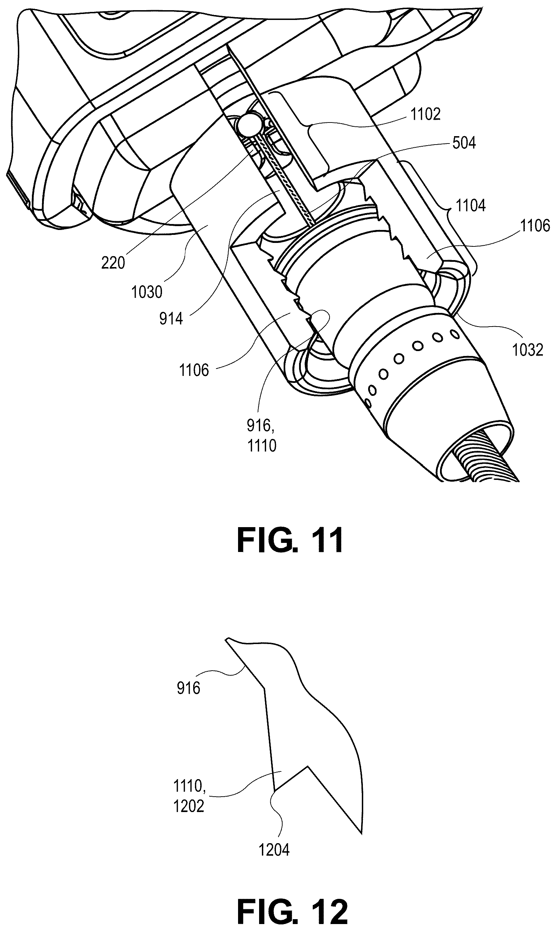

Referring to FIG. 11, a perspective view of a docking cap 320 of a biostimulator transport system 300 inserted into a loading tool 904 is shown in accordance with an embodiment. When the top wall segment 1030 and the bottom wall segment 1032 combine, the loading slot 914 can provide a visual window into the receptacle volume 910. The loading slot 914 allows passage of the tether 504 of the biostimulator transport system 300 into the attachment feature 220 of the biostimulator 100 in the receptacle volume 910. For example, the loading slot 914 can include a distal slot section 1102 through which the ball 502 and the tether 504 pass to load into the locking channel 408 in the attachment feature 220.

The loading slot 914 can also include a proximal slot section 1104, proximal to the distal slot section 1102. The proximal slot section 1104 can allow the docking cap 320 to pass laterally through the loading slot 914 into the receptacle volume 910. To accommodate the various widths of the biostimulator transport system components, the loading slot sections may have corresponding widths. More particularly, the loading slot cutout can match a silhouette of the biostimulator transport system 300, e.g., the docking cap 320 and/or the tether 504 and the ball 502. In an embodiment, the proximal slot section 1104 is wider than the distal slot section 1102. The proximal slot section 1104 can have a width that corresponds to a width of the docking cap 320, and the distal slot section 1102 can have a width that corresponds to a width of the ball 502 and tether 504. The loading slot 914 can therefore allow the coupling mechanism 350 and the docking cap 320 to be intuitively fit into place within the loading tool 904.

The loading tool slot cutout can function to ensure that the biostimulator transport system 300 inserts smoothly and securely into the loading tool 904. For example, the loading tool 904 can have features in a proximal end 221 to guide a user to execute the loading process using a ball-cone catheter method. The features can have an intuitive feel and ergonomic attributes to successfully place the ball 502 into the locking channel 408 and lateral slot 410 in the attachment feature 220. Accordingly, the ball 502 and tether 504 can be secured, and the conical insert 506 can be advanced to lock the biostimulator transport system 300 to the biostimulator 100.

The slot feature can include a taper to guide the docking cap 320 into place when the ball 502, tether 504, and docking cap 320 are loading laterally into the closed loading tool 904. In an embodiment, the receptacle portion 908 includes slot surfaces 1106 facing each other across the proximal slot section 1104. The slot surfaces 1106 can be tapered. The tapered slot surfaces 1106 can be positioned to require an amount of force to push the docking cap 320 into the receptacle volume 910. For example, a lateral distance between the slot surfaces 1106 can be less than a diameter of the docking cap 320, requiring the docking cap 320 to be pressed through the cantilever arms 1034 to snap into place within the receptacle volume 910. The cantilever arms 1034 can have flexibility by virtue of not being directly connected to the semi-cylindrical wall 1036, and can therefore flex outward to permit passage of the docking cap 320. That is, the parting line between the top wall segment 1030 and the bottom wall segment 1032 allows the cantilever arms 1034 to flex independently and to be wedged open by the docking cap 320 during insertion of the docking cap 320 into the loading slot 914. In other words, the tapering surfaces allow the docking cap 320 to slide laterally inward and snap into the receptacle volume 910, ensuring a snug fit and preventing the docking cap 320 from passing radially outward when the biostimulator transport system 300 engages the loading tool 904.

The distal slot section 1102 of the loading slot 914 can be formed in a distal subportion of the receptacle portion 908 of the loading tool 904 that otherwise has a solid cylindrical profile. For example, an outer surface of the receptacle portion 908 extending circumferentially on either side of the distal slot section 1102 can be cylindrical. The solid cylindrical profile can support the cantilever arms 1034 by providing a robust base at which the cantilever arms 1034 extend from. It will be appreciated that the cylindrical profile is more robust, for example, than a tapered profile over the same length would be. Accordingly, the geometry shown in FIG. 11 can support resilient flexing of the cantilever arms 1034 during docking cap 320 insertion.

In an embodiment, the interior surface 916 of the loading tool 904, which surrounds the receptacle volume 910, can include a friction feature 1110. The friction feature 1110 can be a surface morphology or treatment that resists sliding movement of the docking cap 320 relative to the receptacle portion 908. For example, the friction feature 1110 can be a roughening of the interior surface 916 that has an increased friction and therefore resists sliding of the docking cap 320.

Referring to FIG. 12, a detailed view of a friction feature 1110 of a loading tool 904 is shown in accordance with an embodiment. The friction feature 1110 can include several ratchet teeth 1202. The ratchet teeth 1202 can resist movement of the docking cap 320. For example, the ratchet teeth 1202 can be directionally oriented to resist movement of the docking cap 320 in a particular direction. As shown, the ratchet teeth 1202 can be proximally directed, e.g., sloped in a proximal direction along the longitudinal axis 208. The ratchet teeth 1202 slope can extend from the interior surface 916 proximally to a tooth point 1204 that is radially inward from a base of the tooth 1202. The tooth point 1204 can resist distal motion of the docking cap 320 within the receptacle volume 910. The teeth 1202 may, however, permit proximal motion of the docking cap 320. The teeth 1202 may therefore act as a ratchet-slide lock mechanism to secure the docking cap 320 and to allow the docking cap 320 to be retracted proximally to place tension on the tether 504. The ratchet mechanism can secure the docking cap 320 to maintain the tension in the tether 504 when the ball 502 is locked within the locking channel 408.

The ratchet teeth 1202 are one implementation of the friction feature 1110, however, other friction features may be used. For example, the interior surface 916 can include a compressible surface that forms a press fit against the docking cap 320 and therefore resists longitudinal motion of the docking cap 320 within the receptacle portion 908. The biostimulator transport system 300 may nonetheless be pulled back to slide the docking cap 320 and place tension on the tether 504, but the friction feature 1110 can resist subsequent distal motion of the docking cap 320. Accordingly, upon loading the biostimulator transport system 300 into the loading tool 904, the tether 504 can be tensioned between the ball 502 and the docking cap 320 to allow a user to single-handedly advance the locking tube 602 and insert the conical insert 506 into the attachment feature 220. More particularly, the friction feature 1110 can facilitate single-handed operation of loading process.

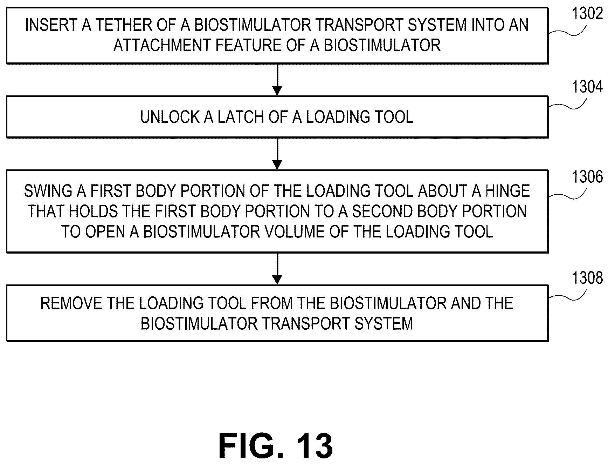

Referring to FIG. 13, a flowchart of a method of loading a biostimulator 100 onto a biostimulator transport system 300 is shown in accordance with an embodiment. The method can be used to lock the biostimulator 100 onto the biostimulator transport system 300 via the ball and tether coupling mechanism. At operation 1302, the tether 504 of the biostimulator transport system 300 is inserted into the attachment feature 220 of the biostimulator 100. The biostimulator 100 can be disposed in the biostimulator volume 1016 of the loading tool 904, with the base 404, neck 402, and head 406 located in the receptacle volume 910, and the biostimulator 100 may therefore be exposed laterally to the surrounding environment through the loading slot 914. In an embodiment, the tether 504 can pass through the loading slot 914 into the receptacle volume 910 to engage the biostimulator 100. For example, the tether 504 can pass through the lateral slot 410 to enter the locking channel 408 of the biostimulator 100.

The insertion process can also include inserting the docking cap 320 of the biostimulator transport system 300 through the loading slot 914 into the receptacle volume 910. For example, as described above, the docking cap 320 can be snapped into the receptacle volume 910 through the cantilever arms 1034. When the docking cap 320 is in the receptacle volume 910, the docking cap 320 can be retracted within the receptacle volume 910 to bear against the friction feature 1110 of the interior surface 916 surrounding the receptacle volume 910. For example, the docking cap 320 can ratchet proximally to engage a set of teeth 1202 that holds the docking cap 320 in place and maintains tension in the tether 504 between the docking cap 320 and the ball 502.

The locking tube 602 may then be advanced over the tensioned tether 504 to wedge the conical insert 506 into the corresponding tapering recess in the proximal end of the head 406. The conical insert 506 can be forced into the recess, and the head 406 can be squeezed between the conical insert 506 and the ball 502. Accordingly, the biostimulator 100 may become locked to the coupling mechanism of the biostimulator transport system 300.

At operation 1304, the latch 1020 of the loading tool 904 can be unlocked. The latch 1020, which may be mounted on the first body portion 1002 of the loading tool 904, can fasten the first body portion 1002 to the second body portion 1004 in the closed configuration. When the latch 1020 is unlocked, the loading tool 904 can transition from the closed configuration to the open configuration.

At operation 1306, the first body portion 1002 can be swung about the hinge 1006 to open the biostimulator volume 1016 of the loading tool 904. The hinge 1006 holds the first body portion 1002 to the second body portion 1004. Accordingly, when the body portions swing apart about the hinge 1006, the body portions can remain connected but can open and release the biostimulator 100.

At operation 1308, the loading tool 904 can be removed from the biostimulator 100 and the biostimulator transport system 300. In the open configuration, the loading tool 904 may simply fall away from the biostimulator 100 and the biostimulator transport system 300, which are locked together. Alternatively, the loading tool 904 may be manually removed from the biostimulator 100 and the biostimulator transport system 300. In either case, the biostimulator 100 may be loaded onto the biostimulator transport system 300 to permit delivery of the biostimulator into a patient anatomy.

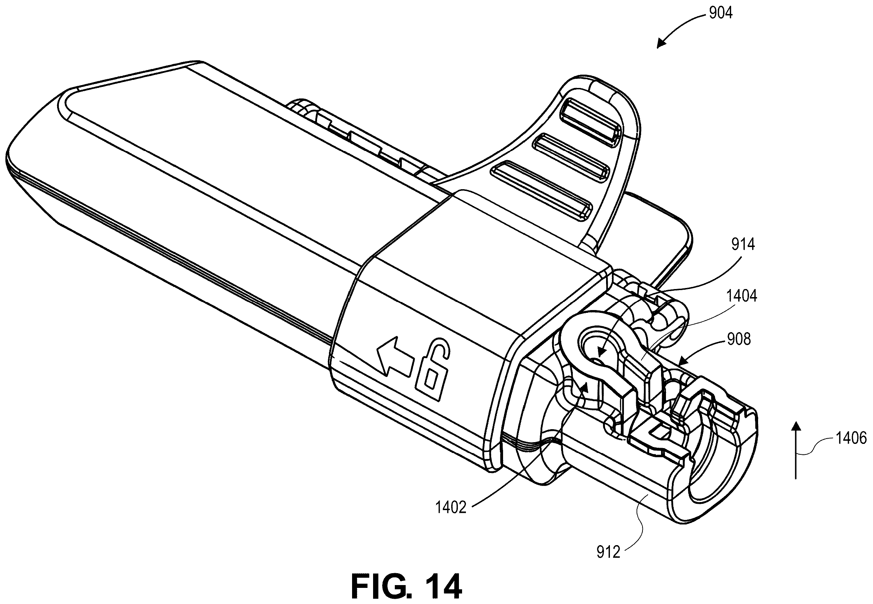

Referring to FIG. 14, a perspective view of a loading tool 904 in a closed configuration is shown in accordance with an embodiment. FIG. 14 illustrates an alternative embodiment of a loading tool 904, similar to the loading tool 904 described with respect to FIGS. 9 and 10. The loading tool 904 includes a receptacle portion 908 that has the loading slot 914 as described above. The receptacle portion 908 can include a receptacle wall 912 that surrounds the loading slot 914, and the receptacle portion 908 may include several features that guide portions of the biostimulator transport system 300 into the loading slot 914. The loading tool 904 and the receptacle portion 908 of the loading tool 904 can define the loading slot 914 in that the receptacle wall 912 surrounds the loading slot 914 and provides a passage into the loading slot 914. The loading tool 904 in FIG. 14 is shown in a closed configuration; however, it can be actuated into an open configuration, as is shown in FIG. 10.

The loading tool 904 can have several features that improve the ability and the ease of loading the biostimulator transport system 300 into the biostimulator 100. In an embodiment, the loading tool 904 includes a funnel 1402 that provides guidance of the ball 502 or the tether 504 into the loading slot 914. The funnel 1402 can be incorporated in the receptacle wall 912 or in the receptacle portion 908. The funnel 1402 can include a funnel wall 1404 which extends in a lateral direction 1406 from the receptacle wall 912. The lateral direction 1406 may, for example, be a vertical direction or a direction that is orthogonal to a central channel or a central axis of the loading slot 914. Accordingly, when the biostimulator transport system 300 is inserted into the funnel 1402, it can be guided in the orthogonal direction until the ball 502 engages the biostimulator 100 that is retained within the loading tool 904 along the central axis.

The funnel 1402 can include a geometry configured to guide the ball 502 and the tether 504 into the loading slot 914. More particularly, the funnel 1402 can include a geometry configured to guide the ball 502 and the tether 504 into a horseshoe-shaped receiving area, providing a visual cue and facilitating alignment. In addition to providing guidance to the biostimulator transport system 300, the funnel 1402 can provide a visual cue to facilitate alignment during loading. More particularly, the funnel 1402 can be viewed by a physician and can provide a target to insert the ball 502. Once inserted into the funnel 1402, the ball 502 can be guided downward into and loaded into the biostimulator 100. Accordingly, in an embodiment, the ball 502 rather than the docking cap 320 may be a primary alignment target. That is, the physician can focus on inserting the ball 502 into the funnel 1402 rather than inserting the docking cap 320 into a particular slot. As a result of this ball-primary design, a broader range of tolerances between the docking cap 320 and the ball 502 position may be accommodated. More particularly, the longitudinal distance between the ball 502 and the docking cap 320 may be less critical to achieving insertion of the ball 502 into the biostimulator 100.

The loading tool 904 can have additional embodiments to receive and guide portions of the biostimulator transport system 300. For example, the loading tool 904 can include an emboss of a locking element silhouette. An emboss of a locking element silhouette, such as a silhouette of the ball 502 or the tether 504 of the biostimulator transport system 300, acts as a guide to feed the biostimulator transport system 300 components into the locking channel 408 and lateral slot 410. The loading tool 904 can also include an emboss of the docking cap 320. The emboss, like the funnel 1402, can have a silhouette of the docking cap 320. The silhouette can be a raised embossment extending in the lateral direction 1406. An emboss of a docking cap silhouette can guide the docking cap 320 when the user pushes the docking cap 320 into the loading slot 914 to initiate the loading process. By pushing the docking cap 320 into the embossment, the docking cap 320 can be guided laterally downward into the loading slot 914 to initiate the loading process. The loading of the docking cap 320 can be done simultaneously with, before, or after inserting the ball 502 into the funnel 1402.

A cutout in the receptacle wall 912, referred to as a flex slot below, provides a cutout between the funnel emboss and the docking cap receptacle to thin out and increase flexibility of the region. More particularly, the cutout creates a bending point between the docking cap receptacle and the funnel 1402. The cutout thins out the material and increases the flexibility of the region. As a result, the region that receives the docking cap 320 can flex laterally, easing docking cap 320 insertion by lowering the required insertion force. For example, the receptacle portion 908 can bend at the cutout. As a result, when the physician loads the docking cap 320 into the cap receptacle, the material can flex, which can provide a more ergonomic insertion and can lower the required insertion force.

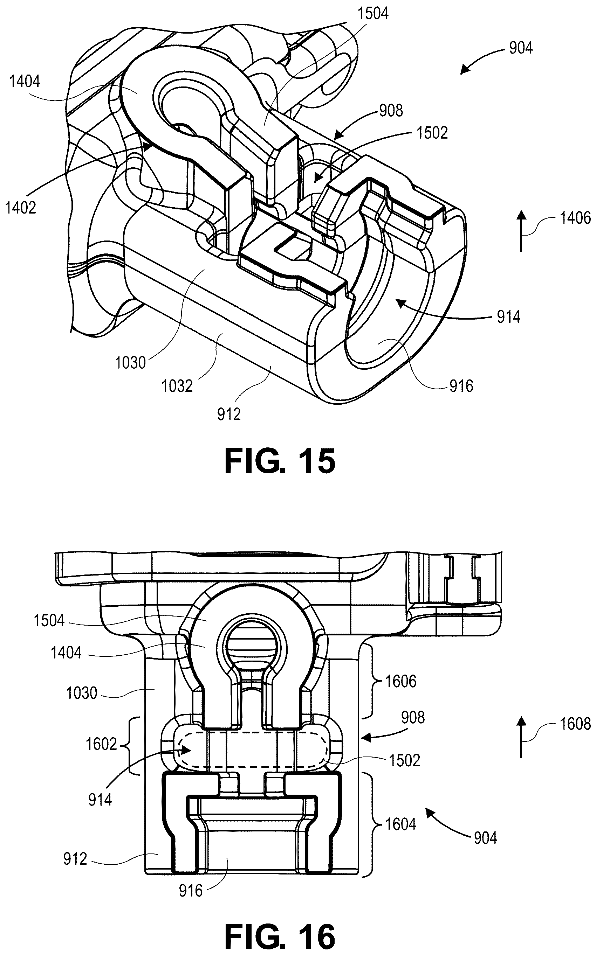

Referring to FIG. 15, a perspective view of a receptacle portion 908 of a loading tool 904 is shown in accordance with an embodiment. FIG. 15 illustrates a detailed perspective view of the receptacle portion 908 of the loading tool 904. As described above, the funnel 1402 can include a funnel wall 1404 that extends in the lateral direction 1406 from the receptacle wall 912. The funnel wall 1404 can be vertical; however, it may have a profile that widens from a proximal to a distal location. The widening and the resulting larger target that it provides for ball 502 insertion is described in more detail below. Importantly, the funnel 1402 is embossed over the loading slot 914, and the funnel 1402 therefore provides a guide in the vertical direction from the surrounding environment into the loading slot 914.

A loading tool 904 includes a distal slot section. The distal slot section includes a funnel 1402 embossed over a loading slot 914. The funnel 1402 includes a funnel wall 1404. The funnel wall 1404 extends in a lateral direction 1406 from a receptacle wall 912. The receptacle wall 912 includes a flex slot region 1602. The funnel wall 1404 geometry can have a compact form factor. In particular, the funnel wall 1404 can have a proximal taper on a top surface 1504. The top surface 1504 can be the surface that is furthest in the lateral direction 1406 from the loading slot 914, and it would be the surface that is initially visible to the physician when attempting to insert the ball 502. The proximal taper of the top surface 1504 of the ball funnel wall 1404 can allow the loading tool 904 to have a more compact form factor and fit within a smaller package. In particular, the top surface can be tapered in the proximal direction, meaning that a distal location on the top surface 1504 can be higher above the receptacle wall 912 than a proximal point on the top surface 1504.

The funnel 1402 can have additional features that improve insertion. By way of example, the funnel 1402 may include a fillet or a chamfer around an edge of the funnel wall 1404. A fillet or chamfer around the edge of the funnel wall 1404 can increase the target area for ball 502 insertion. When the physician inserts the ball 502, it can be guided along the fillet into the vertical slot that is within the funnel 1402. The vertical slot may also be referred to as a funnel slot and may be the space that passes from the surrounding environment into the loading slot 914. When viewing the funnel 1402 downward in the lateral direction 1406 (FIG. 16), the physician can see through the funnel channel toward an interior surface 916 of the receptacle portion 908. When the biostimulator 100 is in the loading tool 904, the view into the funnel channel may also look into the slot within which the ball 502 is passed into the attachment feature 220.

Additional description may be provided related to the distal slot section. The distal slot section in the loading tool 904 can create a flex slot region. As described above, the flex slot region can provide a cutout, referred to as a flex slot 1502, between the docking cap silhouette and the ball-tether silhouette. A cutout is provided between the docking cap silhouette and the ball-tether silhouette to create a spring-like flex mechanism that reduces insertion force and provides smoother tactile feedback during docking cap engagement. This mechanism reduces insertion force and provides smoother tactile feedback during docking cap 320 engagement. The cutout allows the portion of the receptacle portion 908 that receives the docking cap 320 to move relative to the portion that receives the ball 502.

Referring to FIG. 16, a top view of a receptacle portion 908 of a loading tool 904 is shown in accordance with an embodiment. FIG. 16 provides a top view of the receptacle portion 908 in which the distally widening shape of the funnel wall 1404 is visible. In an embodiment, the funnel wall 1404 is hippocrepiform. The hippocrepiform shape may be horseshoe-shaped or U-shaped. The shape can have a width between a leftward and rightward funnel wall 1404 that is narrower at a proximal location than at a distal location where the ball 502 is inserted. Accordingly, the hippocrepiform shape can mimic the profile of the ball 502 and the tether 504, providing a passage through which the ball 502 and the tether 504 can be inserted.

The flex slot region 1602 has the flex slot 1502 in the receptacle wall 912 longitudinally between a lock receptacle region 1606 and a cap receptacle region 1604. The receptacle wall 912 is stiffer in the lock receptacle region 1606 and the cap receptacle region 1604 than the flex slot region 1602. More particularly, and as described above, the flex slot 1502 can thin out the material in the intermediate region by removing material in that section. The flex slot 1502 can therefore allow bending and relative movement between the lock receptacle region 1606 and the cap receptacle region 1604, thereby reducing the insertion force required to load the biostimulator transport system 300 into the loading tool 904. It has been shown that the flex wall slot improves, e.g., reduces, the insertion force required to load the biostimulator transport system 300 into the loading tool 904.

Like the funnel 1402, the cap receptacle can provide a loading slot 914 within the cap receptacle region 1604 that widens in a distal direction 1608. For example, the distance between the embossment walls at a proximal location may be narrower than the distance between the embossment walls at a location where an end of the docking cap 320 is inserted. The loading tool 904 includes a docking cap silhouette region that allows the user to place the docking cap 320 into the feature, whereupon it engages smoothly due to the flex slot 1502. More particularly, the loading tool 904 includes a cap receptacle region 1604 that conforms to a bell-curved profile of the docking cap 320, with a wider opening to accommodate the bell portion and closer alignment along the narrower body. The cap receptacle region 1604 can conform to the bell-curved profile of the docking cap 320, with a wider opening more distally to accommodate the bell portion and a narrower opening more proximally to accommodate the narrower proximal portion of the docking cap 320. The narrower portion may fit more closely to the docking cap 320 and can provide some press fit between the receptacle portion 908 and the docking cap 320 during docking cap insertion.

Surfaces of the loading tool 904 may be color-highlighted, e.g., white or yellow, to provide visual guidance to the user. For example, the top surface 1504 may be color-highlighted to give a good visual distinction and guidance to the physician or user. Marking can be achieved using laser marking or pad printing. Other forms of marking that are commonly used may also be employed.

In an embodiment, the friction force generated by an interference fit between the loading tool prongs in the cap receptacle region 1604 and the docking cap 320 provides sufficient retention force. There may be no need for a ratcheting feature in the embodiment of FIG. 16. Instead, a friction force can be generated by an interference fit between the loading tool prongs and the docking cap 320. The friction force can provide sufficient retention force to the docking cap 320, which can allow for tension to be generated in the tether 504, as described above. The interference fit between the docking cap 320 and the loading tool prongs can be approximately 0.030 inches (0.762 mm). The press fit has sufficient friction to hold the docking cap 320 when axial load has not been applied to the biostimulator transport system 300.

During docking, the user inserts the ball 502 into the horseshoe-shaped receptacle, e.g., the funnel 1402, while the cone, that is, the conical portion or conical insert 506, is recessed within the docking cap 320. After snap-in engagement, the cone, i.e., the conical insert 506, can be advanced forward to complete the docking connection. More particularly, after the ball 502 is inserted and the docking cap 320 is snapped into place, the cone 506 can then be advanced forward to complete the docking connection. Once the ball 502 and the conical insert 506 have inserted into and engaged the attachment feature 220 of the biostimulator 100, the loading tool 904 can be actuated into the open configuration. The loading tool 904 can be opened, and the assembly of the biostimulator 100 and biostimulator transport system 300, e.g., the biostimulator system 902, can be removed from the loading tool 904.

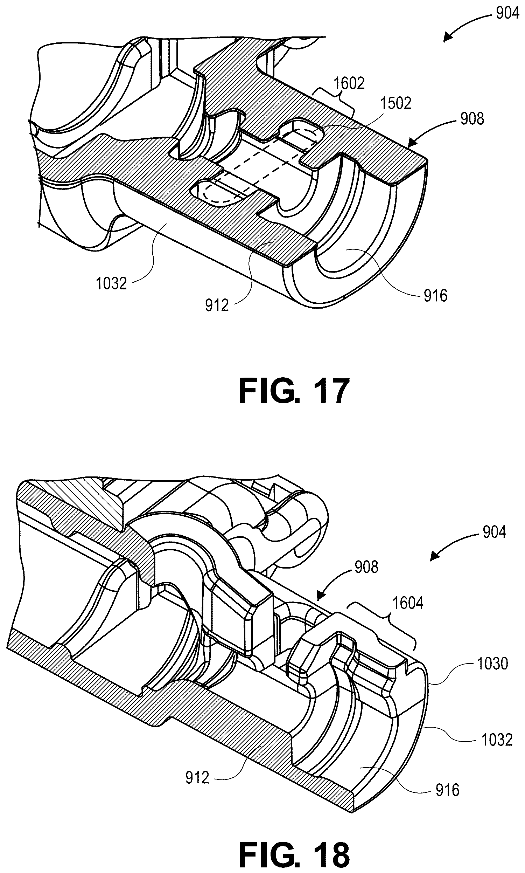

Referring to FIG. 17, a perspective sectional view of a receptacle portion of a loading tool is shown in accordance with an embodiment. The cross-sectional view allows the internal structure of the receptacle portion 908 to be visualized. A sectional view reveals the interior surface 916 of the receptacle wall 912. It also allows for the flex slot 1502 to be seen, and in particular how a thickness of the receptacle wall 912 is thinner in the flex slot region 1602 than it is in the more distal lock receptacle region 1606 or the more proximal cap receptacle region 1604. The different slots and cavities that can be used to, for example, receive the attachment feature 220 or the docking cap 320 are also shown. A combination of structures that are visible can facilitate loading of the biostimulator transport system 300. In particular, the sectional view shows where the ball 502 would be dropped downward into, as well as the insertion location for the docking cap 320, and the channel that interconnects those cavities through the flex slot region 1602. The channel is where the tube of the biostimulator transport system 300 can pass. It can be appreciated that the receptacle portion 908 could flex or bend about the flex slot region 1602.

Referring to FIG. 18, a perspective sectional view of a receptacle portion 908 of a loading tool 904 is shown in accordance with an embodiment. The sectional view provides an alternative perspective sectional view of the receptacle portion 908. The bottom wall segment 1032 is visible, and the view supports additional internal features of the receptacle wall 912 and its relationship to the funnel 1402 geometry. For example, the funnel wall 1404 extending in the lateral direction 1406 upward from the loading slot 914 can be seen. Similarly, the embossment wall within which the docking cap 320 is placed can be seen. The flex slot 1502 that is longitudinally between the funnel wall 1404 and the embossment wall is visible.

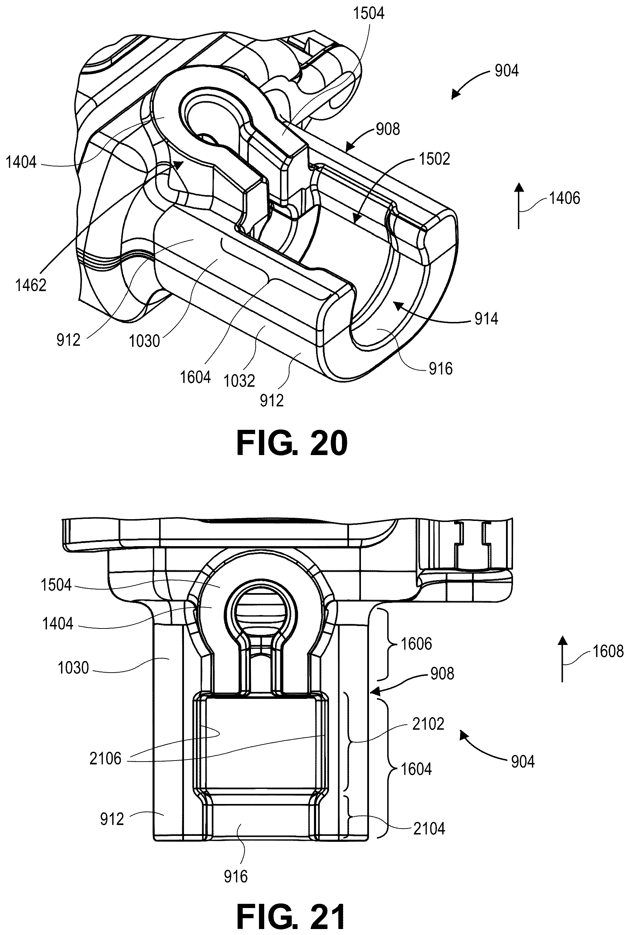

The cap receptacle region 1604 is shown to have a distally widening profile. The widening profile can accommodate various loading positions of the biostimulator transport system 300. More details on this feature are described below, for example, with respect to FIG. 21, but it will be appreciated at this point that the widening cap receptacle can accommodate for the wider distal end of the docking cap 320. It is also notable that there is the top wall segment 1030 and the bottom wall segment 1032. The top wall segment 1030 and the bottom wall segment 1032 are in contact with each other when the loading tool 904 is in the closed configuration. When the loading tool 904 is opened and moved to the open configuration, those walls will separate, allowing for the biostimulator system to be removed from the loading tool 904.

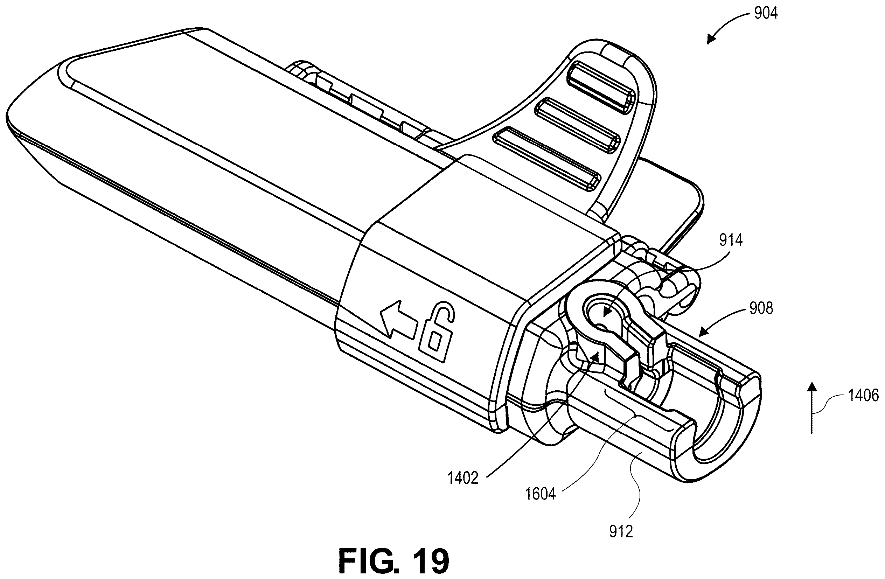

Referring to FIG. 19, a perspective view of a loading tool 904 in a closed configuration is shown in accordance with an embodiment. FIG. 19 illustrates another alternative embodiment of a loading tool 904, similar to the loading tool 904 described with respect to FIGS. 9, 10, and 14. The loading tool 904 includes a receptacle portion 908 that has the loading slot 914 as described above. The receptacle portion 908 can include a receptacle wall 912 that surrounds the loading slot 914, and the receptacle portion 908 may include several features that guide portions of the biostimulator transport system 300 into the loading slot 914. The loading tool 904 and the receptacle portion 908 of the loading tool 904 can define the loading slot 914 in that the receptacle wall 912 surrounds the loading slot 914 and provides a passage into the loading slot 914. The loading tool 904 in FIG. 19 is shown in a closed configuration; however, it can be actuated into an open configuration, as is shown in FIG. 10.

The loading tool 904 can have several features that improve the ability and the ease of loading the biostimulator transport system 300 into the biostimulator 100. In an embodiment, the loading tool 904 includes a funnel 1402 that provides guidance of the ball 502 or the tether 504 into the loading slot 914. The funnel 1402 can be incorporated in the receptacle wall 912 or in the receptacle portion 908. The funnel 1402 can include a funnel wall 1404 which extends in the lateral direction 1406 from the receptacle wall 912. Accordingly, when the biostimulator transport system 300 is inserted into the funnel 1402, it can be guided in the orthogonal direction until the ball 502 engages the biostimulator 100 that is retained within the loading tool 904 along the central axis.

The receptacle portion 908 can also include features relating to receiving and stabilizing the docking cap 320 during the loading process. The receptacle wall 912 can include a cap receptacle region 1604 configured to receive the docking cap 320 of the biostimulator transport system 300. In the embodiment shown in FIG. 19, the cap receptacle region 1604 can have a distally widening profile to accommodate various loading positions of the biostimulator transport system 300. The widening cap receptacle profile can accommodate a wider breadth of distances between the docking cap 320 and the ball 502. For example, as described below, the widened area of the cap receptacle profile can have a length in the axial direction that allows for the bell-shaped docking cap to be engaged at various axial locations. This accommodation can allow for greater manufacturing tolerances and provide more flexibility in the relative positioning of components during the loading process, thereby improving ease of use.

The loading tool 904 can further include a top wall segment 1030 and a bottom wall segment 1032. When the loading tool 904 is in the closed configuration, as shown in FIG. 19, the top wall segment 1030 and the bottom wall segment 1032 can be in contact with each other or in close proximity to define the biostimulator volume within which the biostimulator 100 is held during the loading process. When the loading tool 904 is actuated to the open configuration, the top wall segment 1030 and the bottom wall segment 1032 can separate from each other, allowing the biostimulator 100 and the biostimulator transport system 300 to be removed from the loading tool 904.

Referring to FIG. 20, a perspective view of a receptacle portion 908 of a loading tool 904 is shown in accordance with an embodiment. FIG. 20 illustrates a detailed perspective view of the receptacle portion 908 of the loading tool 904 of the embodiment shown in FIG. 19. As described above, the funnel 1402 can include a funnel wall 1404 that extends in the lateral direction 1406 from the receptacle wall 912. The funnel wall 1404 can be vertical; however, it may have a profile that widens from a proximal to a distal location. The widening and the resulting larger target that it provides for ball insertion is described in more detail above. The funnel 1402 is embossed over the loading slot 914, and the funnel 1402 therefore provides a guide in the vertical direction from the surrounding environment into the loading slot 914.

The receptacle portion 908 in FIG. 20 can include an interior surface 916 that surrounds the loading slot 914 and defines the passage through which the biostimulator transport system 300 is loaded. The interior surface 916 can include a top wall segment 1030 and a bottom wall segment 1032, which come together when the loading tool 904 is in the closed configuration to define the biostimulator volume. The interior surface 916 can be configured to contact the biostimulator 100 and hold the biostimulator 100 in place during the loading process. Similarly, as described in more detail below, the interior surface 916 can have portions that interact with the docking cap 320 when it is inserted into the cap receptacle region 1604.

In the embodiment of FIG. 20, the receptacle wall 912 can include a flex slot 1502 that is integrated with the cap receptacle region 1604. Unlike the embodiment of FIG. 15, in which the flex slot 1502 is a distinct cutout between the funnel 1402 and the cap receptacle region 1604, the embodiment of FIG. 20 combines the flex slot 1502 with the cap receptacle region 1604. More particularly, the widened portion of the cap receptacle region 1604 provides flexibility that eases insertion of the docking cap 320. The distally widening profile of the cap receptacle region 1604 creates a thinner wall section that can flex laterally, thereby lowering the required insertion force when the docking cap 320 is loaded into the receptacle portion 908. Accordingly, the widened cap receptacle region 1604 serves a dual function: it accommodates various loading positions of the biostimulator transport system 300 while also providing the spring-like flex mechanism that reduces insertion force and provides smoother tactile feedback during docking cap engagement.

Referring to FIG. 21, a top view of a receptacle portion 908 of a loading tool 904 is shown in accordance with an embodiment. FIG. 21 provides a top view of the receptacle portion 908 of the loading tool 904 of the embodiment shown in FIGS. 19 and 20. In the top view, the distally widening shape of the funnel 1402 is visible. As described above with respect to FIGS. 19 and 20, in the present embodiment, the flex slot 1502 is integrated with the cap receptacle region 1604 such that the widened portion of the cap receptacle region 1604 provides flexibility that eases insertion of the docking cap 320 while also accommodating various loading positions.

The cap receptacle region 1604 can have a distal receptacle region 2102 and a proximal receptacle region 2104. The distal receptacle region 2102 may include an entry channel between the surrounding environment and the loading slot 914, and the proximal receptacle region 2104 may also provide such a channel. The distal receptacle region 2102 channel, however, may be wider than the proximal receptacle region 2104 channel. Accordingly, the wider distal end of the docking cap 320 may be received within the distal receptacle region 2102. By contrast, the narrower proximal end of the docking cap 320 may be inserted through the proximal receptacle region 2104.

The distal receptacle region 2102 can have a distal end that coincides with a proximal end of the funnel 1402. The transition between the distal receptacle region 2102 and the proximal receptacle region 2104, e.g., a tapered transition between the differently sized channels, may be located between the funnel 1402 and a proximal end of the loading tool 904.

The differently sized receptacle regions can interact differently with portions of the docking cap 320. The retention geometry of the cap receptacle region 1604 provides two-point contact on either side of the docking cap 320 at the wider bell region and line contact along the narrower constricted body, which stabilizes the docking cap 320 and inhibits rotation. For example, the geometry of the distal receptacle region 2102 can provide for a two-point contact 2106 on either side of the docking cap 320. The two-point contact 2106 may be between an edge of the distal receptacle region 2102 and an outer surface of the wider bell region of the docking cap 320. When the docking cap 320, which has the bell-shaped profile, is inserted into the distal receptacle region 2102, two points on the docking cap 320 can contact corresponding contact points 2106 on the distal receptacle region 2102. The contacts at the contact points 2106 can create a friction fit between the docking cap 320 and the loading tool 904 that stabilizes the docking cap 320. The friction may be limited in surface area, however, to allow for easier insertion and good tactile feel when the docking cap 320 is pressed into place.

By contrast to the limited-length contact points 2106 of the distal receptacle region 2102, the narrower proximal receptacle region 2104 may provide a larger contact area between the loading tool 904 and the docking cap 320. The narrower constricted body of the proximal receptacle region 2104 may create a line of contact around a circumference or a substantial portion of a circumference of the docking cap 320. The increased contact area can generate higher friction over the proximal receptacle region 2104 than within the distal receptacle region 2102. The friction can stabilize the docking cap 320, and furthermore, the friction can resist axial movement of the docking cap 320 when it is inserted into the loading tool 904. Accordingly, the user can pull the biostimulator transport system 300 to generate tension in the tether 504 between the ball 502 and the docking cap 320 after insertion into the loading tool 904. The friction can retain the tensioned state even after the user releases the biostimulator transport system 300.

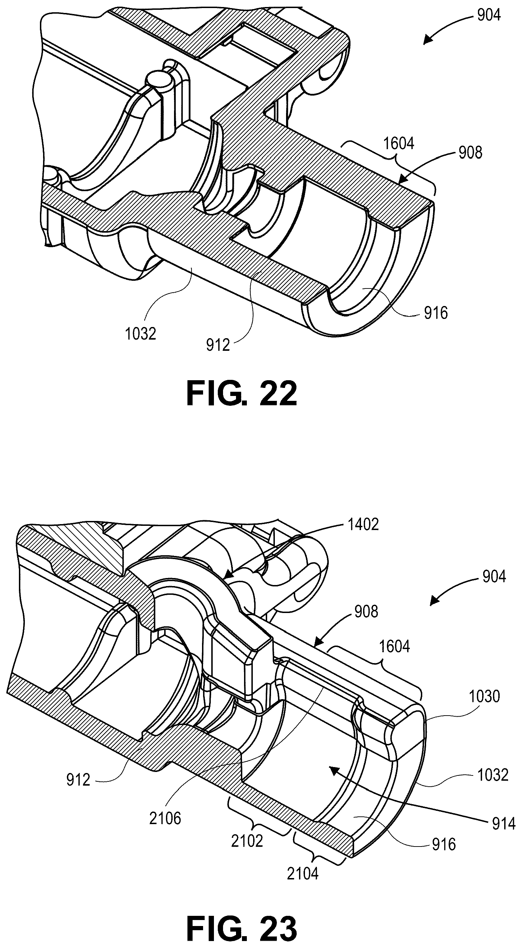

Referring to FIG. 22, a perspective sectional view of a receptacle portion 908 of a loading tool 904 is shown in accordance with an embodiment. The cross-sectional view allows the internal structure of the receptacle portion 908 to be visualized. A sectional view reveals the interior surface 916 of the receptacle wall 912. Unlike the embodiment of FIG. 17, which includes a distinct flex slot 1502, the embodiment of FIG. 22 does not include a separate flex slot. Instead, the receptacle wall 912 has a thinner wall over a longer length of the distal receptacle region 2102. The thinner wall in the distal receptacle region 2102 provides the flexibility that eases insertion of the docking cap 320, as described above.

The sectional view of FIG. 22 reveals that the distal receptacle region 2102 has a wall that is thicker than the proximal receptacle region 2104 wall. The different wall thicknesses in the distal receptacle region 2102 and the proximal receptacle region 2104 can correspond to the different contact geometries described above with respect to FIG. 21. For example, the thicker wall of the distal receptacle region 2102 can provide the two-point contact 2106 with the wider bell region of the docking cap 320, while the thinner wall of the proximal receptacle region 2104 can conform more closely to the narrower body of the docking cap 320 to provide the line contact that generates higher friction.

The different slots and cavities that can be used to, for example, receive the attachment feature 220 or the docking cap 320 are also shown in FIG. 22. A combination of structures that are visible can facilitate loading of the biostimulator transport system 300. In particular, the sectional view shows where the ball 502 would be dropped downward into, as well as the insertion location for the docking cap 320 in the cap receptacle region 1604, and the channel that interconnects those cavities. The channel is where the tube of the biostimulator transport system 300 can pass. It can be appreciated that the receptacle portion 908 could flex or bend at the thinner wall section of the distal receptacle region 2102.

Referring to FIG. 23, a perspective sectional view of a receptacle portion 908 of a loading tool 904 is shown in accordance with an embodiment. The sectional view provides an alternative perspective sectional view of the receptacle portion 908 of the loading tool 904 of the embodiment shown in FIGS. 19-22. The bottom wall segment 1032 is visible, and the view supports additional internal features of the receptacle wall 912 and its relationship to the funnel 1402 geometry. For example, the funnel 1402 extending in the lateral direction upward from the loading slot 914 can be seen. Similarly, the cap receptacle region 1604 within which the docking cap 320 is placed can be seen.

The sectional view of FIG. 23 reveals the interior surface 916 of the receptacle wall 912 and the relationship between the distal receptacle region 2102 and the proximal receptacle region 2104 of the cap receptacle region 1604. As described above with respect to FIG. 21, the distal receptacle region 2102 has a wider channel than the proximal receptacle region 2104 to accommodate the bell-shaped profile of the docking cap 320. The contact points 2106 of the distal receptacle region 2102, which provide the two-point contact with the docking cap 320, can be visualized in the sectional view. Similarly, the narrower proximal receptacle region 2104, which provides the line contact with the docking cap 320 for increased friction and stabilization, can be seen.

The cap receptacle region 1604 is shown to have the distally widening profile described above with respect to FIGS. 19-22. The widening profile can accommodate various loading positions of the biostimulator transport system 300 and can accommodate a wider breadth of distances between the docking cap 320 and the ball 502. Additionally, the thinner wall of the distal receptacle region 2102 provides the flexibility that eases insertion of the docking cap 320, as described above. It is also notable that the top wall segment 1030 and the bottom wall segment 1032 are visible. The top wall segment 1030 and the bottom wall segment 1032 are in contact with each other when the loading tool 904 is in the closed configuration. When the loading tool 904 is opened and moved to the open configuration, those walls will separate, allowing for the biostimulator system 902 to be removed from the loading tool 904.

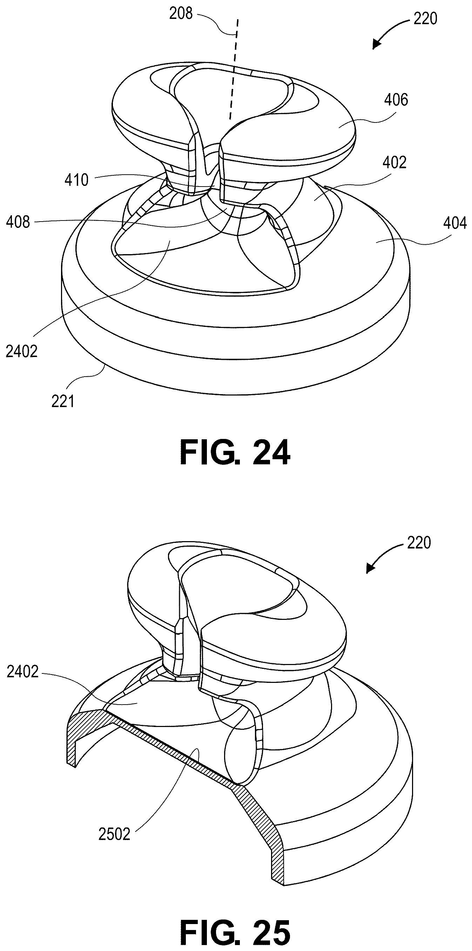

Referring to FIG. 24, a perspective view of an attachment feature 220 of a biostimulator 100 is shown in accordance with an embodiment. The attachment feature 220 can be shaped to receive the release mechanism 350, e.g., the locking ball 502 and/or tether 504, to facilitate engagement between the biostimulator 100 and the biostimulator transport system 300. In an embodiment, the attachment feature 220 is mounted on a proximal end 221 of the housing 206 and extends proximally along the longitudinal axis. The attachment feature 220 can include features to allow components of the biostimulator transport system 300, such as the locking ball 502 and/or tether 504, to engage and connect to the biostimulator 100.

The attachment feature 220 can include a neck 402 that extends proximally from the housing 206. More particularly, the neck 402 can be a portion of the attachment feature 220 that extends from a base 404 at the proximal end 221 to a head 406 of the attachment feature 220 at a most proximal location. The base 404, neck 402, and head 406 can combine to form an overall volume of the attachment feature 220. Within such volume there may be one or more channels to receive components of the biostimulator transport system 300. The channel(s) can include a locking channel 408 within which the locking ball 502 and/or tether 504 can fit.

In an embodiment, the attachment feature 220 includes the locking channel 408 extending longitudinally through the neck 402. A cross-sectional area of the locking channel 408 can vary in the longitudinal direction. For example, the locking channel 408 may be wider at a distal location that receives the ball 502 of the biostimulator transport system 300.

The attachment feature 220 can also include a lateral slot 410 extending laterally, e.g., transverse to the longitudinal direction, to the locking channel 408. The lateral slot 410 can, for example, extend laterally through the neck 402. Accordingly, the lateral slot 410 can form a window into the locking channel 408 that extends along the longitudinal axis. More particularly, the lateral slot 410 can provide a lateral opening through which the tether 504 can pass when the biostimulator transport system 300 is being loaded into the biostimulator 100.

In an embodiment, the locking channel 408 can have a ramped surface. The ramped surface can be configured to cause any forward movement of the ball 502 to translate into lateral displacement. More particularly, when the ball 502 moves in a forward, e.g., distal, direction within the locking channel 408, the ramped surface can redirect the ball 502 movement laterally toward the lateral slot 410. The ramped surface can therefore facilitate exit of the ball 502 and the tether 504 from the locking channel 408 and through the lateral slot 410.

The locking channel 408 can reduce in width distally, as described above, and may also narrow in the lateral direction. More particularly, the locking channel 408 can be defined by a ramped surface 2402 that has a contour to guide the ball 502 laterally inward. The ramped surface 2402 can be located in the locking channel 408 at a position distal to where the ball 502 is seated when the biostimulator transport system 300 is locked to the biostimulator 100. For example, the ramped surface 2402 can be positioned such that if the ball 502 is pushed distally beyond its normal seated position, the ball 502 will contact the ramped surface 2402 and be directed laterally toward the lateral slot 410.

The ramped surface 2402 can have an inclined profile that slopes from a more distal location toward the lateral slot 410. Accordingly, the ramped surface can guide the ball 502 out of the locking channel 408 through the lateral slot 410, facilitating release of the biostimulator transport system 300 from the biostimulator 100 when desired. Similarly, when seating the ball 502, the ball can ride over the ramped surface 2402 in the distal and lateral direction to fit into the neck 402. Accordingly, the ramped surface can guide the ball 502 into the locking channel 408 through the lateral slot 410, facilitating locking of the biostimulator transport system 300 to the biostimulator 100 when desired.

Referring to FIG. 25, a perspective sectional view of an attachment feature 220 of a biostimulator 100 is shown in accordance with an embodiment. The sectional view illustrates a cross-section of the attachment feature 220 at a lateral position near an exterior of the attachment feature 220. The ramped surface 2402 can include a ramp profile 2502, which may be a lower edge of the ramped surface 2402. The ramp profile 2502 at a more lateral position, such as that shown in FIG. 25, may have a larger average radius of curvature. For example, the ramp profile 2502 at a cross-sectional plane near a plane that is coincident with the outer cylindrical portion of the base 404 may be nearly a straight line. As a result, the average radius of curvature can be very large, e.g., near infinity. The larger radius of curvature allows for a larger profile area within which the ball 502 may be inserted into the locking channel 408 through the lateral slot 410.

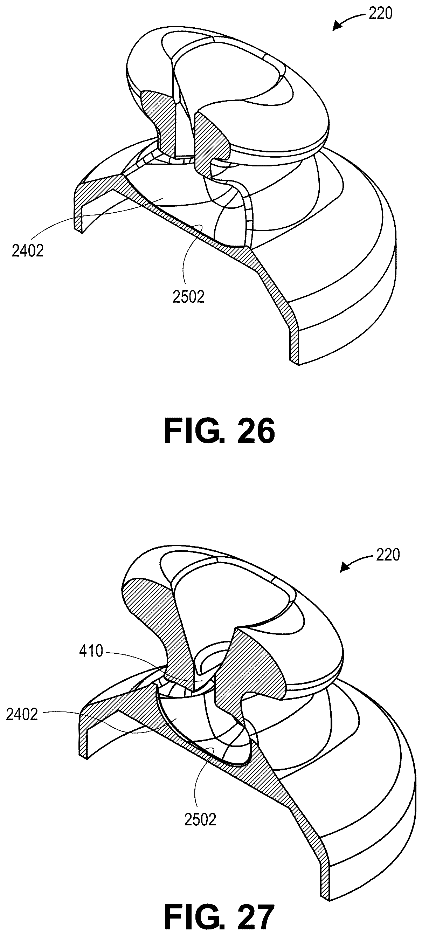

Referring to FIG. 26, a perspective sectional view of an attachment feature 220 of a biostimulator 100 is shown in accordance with an embodiment. The sectional view illustrates a second lateral cross-section that is more central than the cross-section shown in FIG. 25. At this more central location, the ramp profile 2502 may have a smaller average radius of curvature than the ramp profile 2502 shown in FIG. 25. For example, a central portion of the ramp profile 2502 may be nearly straight; however, the outer edges of the ramp profile 2502 may begin to curve upward. The curvature at the edges can decrease the average radius of curvature. Accordingly, the average radius of curvature of the ramp profile 2502 may decrease as the ramp profile 2502 nears a centerline of the attachment feature 220.

Referring to FIG. 27, a perspective sectional view of an attachment feature 220 of a biostimulator 100 is shown in accordance with an embodiment. Similar to FIG. 26, the ramp profile 2502 may continue to decrease in average radius of curvature at a cross-section that is nearer to the centerline of the attachment feature 220. A majority of the ramp profile 2502 may be curved at this radially inward location; however, a smaller portion of the ramp profile 2502 may still be straight. Nonetheless, the average radius of curvature of the ramp profile 2502 in FIG. 27 can be smaller than that of FIG. 26.

It will be appreciated that, as the radius of curvature decreases in the inward lateral direction, the ramped surface 2402 can guide the ball 502 toward a more central position of the attachment feature 220. For example, the ball 502 may be guided downward along the ramp profile 2502 toward the straight segment. The straight segment can be radially aligned with the lateral slot 410. Therefore, the ramp profile 2502 can act as a guide to direct the ball 502 into place within the locking channel 408.

It is also noteworthy that as the cross-sections are taken at more and more laterally central locations, the ramp profile 2502 may move in a proximal direction. For example, the ramp profile 2502 in FIG. 25 may be at a more distal location than the ramp profile 2502 in FIG. 27. The ramp profile 2502 in FIG. 26 may be at an intermediate axial location as compared to FIGS. 25 and 27. The proximally progressing ramp profile 2502 is a characteristic of the proximally tapering ramped surface 2402. That is, the ramped surface 2402 can ramp or taper in the proximal direction and in the laterally inward direction. Therefore, each cross-section that is taken in the radial direction presents the ramp profile 2502 at a more and more proximal location.

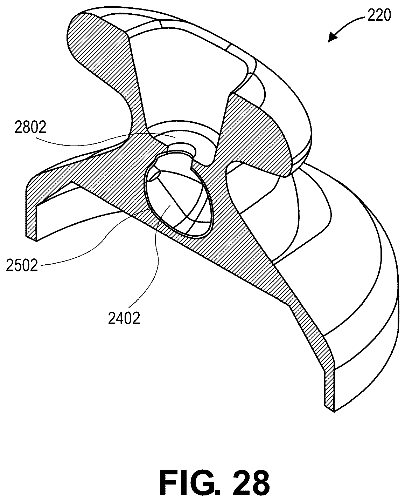

Referring to FIG. 28, a perspective sectional view of an attachment feature 220 of a biostimulator 100 is shown in accordance with an embodiment. The sectional view illustrates a central location where the cross-section coincides with a central axis of the attachment feature 220. At this central location, the ramp profile 2502 may be mostly curved. More particularly, the average radius of curvature of the ramp profile 2502 at the central location may be smaller than the ramp profile 2502 shown in FIGS. 25-27. The ramp profile 2502 may be nearly circular and can approximate a diameter of the ball 502.

The attachment feature 220 can include a locking wall 2802. The locking wall 2802 can be a wall segment of the attachment feature 220 that defines a portion of the locking channel 408. In an embodiment, the ball 502 can fit within the ramp profile 2502 and the tether 504 can extend proximally through a hole in the locking wall 2802. When the ball 502 and the tether 504 are fit within the ramp profile 2502 and passing through the locking wall 2802, the ball 502 can interfere with the locking wall 2802 to lock the biostimulator transport system 300 to the attachment feature 220. Accordingly, the ramped surface 2402 and the ramp profile 2502 can guide the ball 502 into a final resting and locking position within the locking channel 408, facilitating secure engagement between the biostimulator 100 and the biostimulator transport system 300.

In the foregoing specification, the invention has been described with reference to specific exemplary embodiments thereof. It will be evident that various modifications may be made thereto without departing from the broader spirit and scope of the invention as set forth in the following claims. The specification and drawings are, accordingly, to be regarded in an illustrative sense rather than a restrictive sense.

## Claims

1. A loading tool (904) for loading a biostimulator (100) onto a biostimulator transport system (300), comprising:
a body portion (906) having a biostimulator volume (1016) configured to receive the biostimulator (100), a receptacle portion (908) extending from the body portion (906), wherein the receptacle portion (908) has a receptacle wall (912) extending around a receptacle volume (910) configured to receive the biostimulator transport system (300), wherein the receptacle volume (910) is in fluid communication with the biostimulator volume (1016), and wherein a loading slot (914) extends laterally through the receptacle wall (912) into the receptacle volume (910).

2. The loading tool of claim 1, wherein the body portion (906) includes a first body portion (1002) hinged to a second body portion (1004), and wherein when a first face (1008) of the first body portion (1002) is apposed to a second face (1010) of the second body portion (1004), a first recess in the first body portion (1002) and a second recess in the second body portion (1004) combine to form the biostimulator volume (1016).

3. The loading tool of claim 1 or 2, wherein the loading slot (914) is configured to allow passage of a tether (504) of the biostimulator transport system (300) into an attachment feature (220) of the biostimulator (100) in the receptacle volume (910).

4. The loading tool of any one of claims 1-3, wherein the loading slot (914) includes a distal slot section (1102) and a proximal slot section (1104), and wherein the proximal slot section (1104) is wider than the distal slot section (1102).

5. The loading tool of claim 4, wherein the receptacle portion (908) includes slot surfaces (1106) facing each other across the proximal slot section (1104), and wherein the slot surfaces (1106) are tapered.

6. The loading tool of claim 4 or 5, wherein the distal slot section (1102) includes a funnel (1402) embossed over the loading slot (914), wherein the funnel (1402) includes a funnel wall (1404) extending in a lateral direction (1406) from the receptacle wall (912).

7. The loading tool of claim 6, wherein the funnel wall (1404) is hippocrepiform.

8. The loading tool of any one of claims 1-6, wherein the receptacle wall (912) includes a flex slot region (1602) having a flex slot (1502) in the receptacle wall (912) longitudinally between a lock receptacle region (1606) and a cap receptacle region (1604), and wherein the receptacle wall (912) is stiffer in the lock receptacle region (1606) and the cap receptacle region (1604) than the flex slot region (1602).

9. The loading tool of claim 8, wherein the loading slot (914) within the cap receptacle region (1604) widens in a distal direction.

10. The loading tool of any one of claims 1-9, wherein the receptacle wall (912) includes an interior surface (916) surrounding the receptacle volume (910), and wherein the interior surface (916) includes a friction feature (1110).

11. The loading tool of claim 10, wherein the friction feature (1110) includes a plurality of ratchet teeth (1202), and wherein the ratchet teeth (1202) are proximally directed.

12. The loading tool of any one of claims 1-11, wherein the receptacle portion (908) includes a plurality of cantilever arms (1034) extending proximally on laterally opposite sides of the loading slot (914).

13. The loading tool of claim 12, wherein the receptacle portion (908) includes a top wall segment (1030) having the plurality of cantilever arms (1034) , and a bottom wall segment (1032) having a semi-cylindrical wall (1036).

14. A biostimulator system (902), comprising:
a biostimulator (100) including a housing (206) containing circuitry, a fixation element (106) coupled to a distal end (215) of the housing (206), and an attachment feature (220) coupled to a proximal end (221) of the housing (206); and
the loading tool (904) of any one of claims 1-14, wherein the biostimulator volume (1016) contains the biostimulator (100).

15. A method of loading a biostimulator (100) onto a biostimulator transport system (300), comprising:
inserting a tether (504) of the biostimulator transport system (300) into an attachment feature (220) of the biostimulator (100), wherein the biostimulator (100) is disposed in a receptacle volume (910) of a loading tool (904), and wherein the tether (504) passes laterally through a loading slot (914) into the receptacle volume (910);
unlocking a latch of the loading tool (904), wherein the latch is mounted on a first body portion (1002) of the loading tool (904), and wherein the latch fastens the first body portion (1002) to a second body portion (1004);
swinging the first body portion (1002) about a hinge that holds the first body portion (1002) to the second body portion (1004) to open a biostimulator volume (1016) of the loading tool (904); and
removing the loading tool (904) from the biostimulator (100) and the biostimulator transport system (300).
